(19) **Europäisches Patentamt / European Patent Office / Office européen des brevets**

(11) **EP 3 134 376 B1**

(12) **EUROPÄISCHE PATENTSCHRIFT**

(45) Veröffentlichungstag und Bekanntmachung des Hinweises auf die Patenterteilung:
**28.02.2018 Patentblatt 2018/09**

(21) Anmeldenummer: **15716549.9**

(22) Anmeldetag: **20.04.2015**

(51) Int Cl.:
*C07C 5/10* (2006.01)    *C07C 5/29* (2006.01)
*C07C 13/18* (2006.01)

(86) Internationale Anmeldenummer:
**PCT/EP2015/058526**

(87) Internationale Veröffentlichungsnummer:
**WO 2015/162097 (29.10.2015 Gazette 2015/43)**

(54) **VERFAHREN ZUR HERSTELLUNG VON CYCLOHEXAN AUS BENZOL UND METHYLCYCLOPENTAN MIT VORGESCHALTETER BENZOLHYDRIERUNG**

METHOD FOR THE PREPARATION OF CYCLOHEXANE FROM BENZENE AND METHYLCYCLOPENTANE WITH UPSTREAM BENZENE HYDROGENATION

PROCÉDÉ DE FABRICATION DE CYCLOHEXANE À PARTIR DE BENZÈNE ET MÉTHYLCYCLOPENTANE PAR HYDROGÉNATION DU BENZÈNE EN AVAL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.04.2014 EP 14165503**

(43) Veröffentlichungstag der Anmeldung:
**01.03.2017 Patentblatt 2017/09**

(73) Patentinhaber: **BASF SE**
**67056 Ludwigshafen am Rhein (DE)**

(72) Erfinder:
• **HÜBNER, Michael**
**69469 Weinheim (DE)**
• **SCHULZ, Lukas**
**68161 Mannheim (DE)**

• **CZAJKA, Pawel**
**68161 Mannheim (DE)**
• **GOBIN, Oliver Christian**
**67065 Ludwigshafen (DE)**
• **HOLUB, Nicole**
**68167 Mannheim (DE)**
• **PFEIFFER, Daniel**
**67435 Neustadt (DE)**
• **WEICKGENANNT, Andreas**
**68309 Mannheim (DE)**
• **PORTA GARCIA, Marta**
**68165 Mannheim (DE)**

(56) Entgegenhaltungen:
EP-A1- 1 995 297    US-A- 2 846 485
US-A- 3 233 001    US-A- 3 309 411
US-A- 3 311 667

**Beschreibung**

Die vorliegende Erfindung betrifft ein Verfahren zur Herstellung von Cyclohexan aus Methylcyclopentan (MCP) und Benzol. Im Rahmen der vorliegenden Erfindung sind MCP und Benzol Bestandteile eines Kohlenwasserstoffgemisches (KG1), das darüber hinaus Dimethylpentane (DMP), gegebenenfalls Cyclohexan und gegebenenfalls mindestens eine Verbindung (Leichtsieder) ausgewählt aus nichtzyklischen $C_5$-$C_6$-Alkanen und Cyclopentan enthält. Zunächst wird Benzol in einem Hydrierungsschritt zu (dem im Kohlenwasserstoffgemisch (KG2) enthaltenen) Cyclohexan umgesetzt, während MCP in Gegenwart eines Katalysators, bevorzugt einer sauren ionischen Flüssigkeit, zu Cyclohexan isomerisiert wird. Nach der Hydrierung, aber vor der Isomerisierung, erfolgt eine Abtrennung der Dimethylpentane (DMP), wobei das im Kohlenwasserstoffgemisch (KG2) enthaltene Cyclohexan zunächst gemeinsam mit DMP abgetrennt wird. Dieses bereits vor der Isomerisierung vorhandene Cyclohexan kann von DMP in einem nachgeschalteten Rektifikationsschritt wieder abgetrennt und isoliert und/oder in das Verfahren zur Cyclohexanherstellung rückgeführt werden. Zwischen DMP-Abtrennung und MCP-Isomerisierung erfolgt - sofern Leichtsieder im Kohlenwasserstoffgemisch (KG1) enthalten - gegebenenfalls eine Leichtsiederabtrennung. Im Anschluss an die Isomerisierung erfolgt die Isolierung des Cyclohexans gegebenenfalls unter Rückleitung von nicht isomerisiertem MCP und gegebenenfalls von Leichtsiedern. Vorzugsweise sind im Kohlenwasserstoffgemisch (KG1) Cyclohexan und/oder Leichtsieder enthalten, so dass vorzugsweise eine Leichtsiederabtrennung zwischen DMP-Abtrennung von Isomerisierung durchgeführt wird. Weiterhin ist es bevorzugt, dass zusätzlich eine Abtrennung des Cyclohexans von DMP durchgeführt wird, also der Cyclohexan-Anteil, der bei der Benzol-Hydrierung anfällt sowie gegebenenfalls im Ausgangsgemisch (KG1) enthalten ist, isoliert und somit wiedergewonnen wird.

Cyclohexan ist ein wichtiges Wertprodukt der chemischen Industrie, das vorzugsweise durch Hydrierung von in weitgehend reiner Form bereitgestelltem Benzol hergestellt wird. Dabei stellt sich jedoch das Problem, dass das Benzol ein knappes Produkt ist und dass daher mit der Hydrierung zu Cyclohexan andere Verwendungen wie z.B. die Herstellung von Styrol konkurrieren. Daher besteht ein Anreiz, ein Herstellungsverfahren für Cyclohexan zu finden, dass von einem anderen Einsatzstoff als Rein-Benzol ausgeht.

Weiterhin ist es bekannt, dass Cyclohexan nicht nur durch Hydrierung von Benzol, sondern auch durch Isomerisierung von MCP hergestellt werden kann. Als Katalysatoren für eine derartige Isomerisierung werden bevorzugt saure Katalysatoren im Sinne einer Lewisoder Brönstedt-Säure eingesetzt wie z.B. Friedel-Crafts-Katalysatoren oder auch saure ionische Flüssigkeiten.

Die zur Cyclohexanherstellung einsetzbaren Edukte Benzol und MCP sind in der Praxis häufig Bestandteile von Kohlenwasserstoffgemischen. Die konkrete Zusammensetzung der Kohlenwasserstoffgemische kann stark variieren, häufig enthalten sie auch Dimethylpentane (DMP). Darüber hinaus können diese Kohlenwasserstoffgemische auch bereits das eigentliche Zielprodukt Cyclohexan enthalten.

Um jedoch ein reines Zielprodukt, also spezifikationsgerechtes Cyclohexan zu erhalten, muss das Cyclohexan von allen sonstigen im eingesetzten Kohlenwasserstoffgemisch enthaltenen Komponenten, die nach der Hydrierung bzw. Isomerisierung noch vorhanden sind, abgetrennt werden, also auch von im Ausgangsgemisch enthaltenen DMP. Die Abtrennung der DMP vom eigentlichen Verfahrensprodukt Cyclohexan ist jedoch technisch ziemlich anspruchsvoll und aufwändig, insbesondere wenn es sich um das DMP-Isomer 2,4-Dimethylpentan (2,4-DMP) handelt. Der Normalsiedepunkt von 2,4-DMP ist mit 80,52 °C dem Normalsiedepunkt von Cyclohexan (80,78 °C) sehr ähnlich, die Normalsiedepunkte der anderen DMP-Isomere weisen hingegen einen größeren Abstand zu Cyclohexan auf (2,3-DMP hat beispielsweise einen Normalsiedepunkt von 89,88 °C).

US-A 2003/0109767 offenbart ein Verfahren zur Isomerisierung von $C_5$-$C_8$-Paraffinkohlenwasserstoffen (Paraffinen) in Gegenwart einer ionischen Flüssigkeit als Katalysator. Die ionische Flüssigkeit umfasst als Kationen stickstoffhaltige Heterocyclen oder stickstoffhaltige Aliphate, die entsprechenden Anionen sind von Metallhalogeniden abgeleitet. Bei den zu isomerisierenden Paraffinen handelt es sich um lineare Alkane wie n-Hexan oder n-Octan sowie monosubstituierte Alkane wie 3-Methylhexan bzw. Mischungen davon. Durch das in US-A 2003/0109767 beschriebene Verfahren sollen Paraffine mit einem höheren Verzweigungsgrad hergestellt werden. Im Gegensatz dazu hat beispielsweise Cyclohexan gegenüber MCP einen geringeren Verzweigungsgrad. Weiterhin sind in US-A 2003/0109767 keine Angaben dahingehend enthalten, dass im Ausgangsgemisch etwaig enthaltene Aromaten vor der Isomerisierung hydriert werden. In US-A 2003/0109767 ist weiterhin nicht beschrieben, dass das zur Isomerisierung eingesetzte Material auch DMP enthalten kann. Folglich sind in diesem Dokument auch keine Angaben enthalten, an welcher Stelle DMP von Cyclohexan abgetrennt wird bzw. dass diese Abtrennung problematisch ist.

In dem in EP-A 1 403 236 beschriebenen Isomerisierungsverfahren soll ebenfalls ein höherer Verzweigungsgrad in den in Gegenwart einer ionischer Flüssigkeit zu isomerisierenden Paraffinen (Kohlenwasserstoffen) erhalten werden. Das Isomerisierungsverfahren wird zudem in Gegenwart von zyklischen Kohlenwasserstoffen als Additiven und in einem Reaktionsmedium durchgeführt, wobei die zyklischen Kohlenwasserstoffe ein tertiäres Kohlenstoffatom als Struktureinheit enthalten bzw. durch das Reaktionsmedium in eine entsprechende Verbindung mit einer solchen Struktureinheit überführt werden. Vorzugsweise werden Methylcyclohexan oder Dimethylcyclopentan als solche zyklischen Kohlenwasserstoffadditive eingesetzt. Als zu isomerisierende Paraffine werden lineare Alkane wie n-Butan oder n-Octan sowie monomethylsubstituierte Alkane wie 2-Methylhexan eingesetzt. Die ionischen Flüssigkeiten basieren vorzugsweise auf stickstoffhaltigen Heterocyclen oder stickstoffhaltigen Aliphaten als Kationen sowie auf anorganischen Anionen wie Aluminiumhalogeniden. In EP-A 1 403 236 sind ebenfalls keine Angaben enthalten, dass im Ausgangsgemisch etwaig enthaltene Aromaten vor der Isomerisierung hydriert werden. Sinngemäßes trifft auch auf eine etwaige Anwesenheit von DMP im Ausgangsgemisch zu.

US-A 2005/0082201 offenbart ein Verfahren zur Herstellung von Benzin mit einem niedrigen Benzolgehalt, wobei zunächst in einem ersten Verfahrensschritt ein Kohlenwasserstoffgemisch, das Benzol, Olefine und schwefelhaltige Verbindungen wie Thiophene enthält, in eine Rektifikationskolonne eingespeist wird, aus der über Kopf die leichtsiedenden Verbindungen, über einen Seitenabzug eine benzolhaltige Fraktion und aus dem Kolonnensumpf die Schwersieder abgetrennt werden. In einer zweiten Verfahrensstufe wird die aus dem Seitenabzug gewonnene Fraktion in Gegenwart eines Hydrierkatalysators hydriert, wobei Benzol zu Cyclohexan und die Thiophene in Schwefelwasserstoff überführt werden. Das bei der zweiten Verfahrensstufe anfallende cyclohexanhaltige Gemisch eignet sich zur Herstellung von Benzin mit einem niedrigen Benzolgehalt. Eine Isolierung des darin enthaltenen Cyclohexans oder eine Isomerisierung generell, beispielsweise von MCP zu Cyclohexan, sind in US-A 2005/0082201 nicht offenbart. Sinngemäßes trifft auch auf eine etwaige Anwesenheit von DMP im Ausgangsgemisch zu.

WO 2010/027987 betrifft ein weiteres Verfahren zur Verringerung der Konzentration an Benzol in einem kohlenwasserstoffhaltigen Gemisch. In einer ersten Trennstufe wird eine benzolhaltige Fraktion, die Benzol und andere $C_6$-Kohlenwasserstoffe umfasst, von einer Schwersiederfraktion abgetrennt, die Kohlenstoffe mit sieben und mehr Kohlenstoffatomen umfasst. Die benzolhaltige Fraktion wird anschließend hydriert unter Erhalt einer Kohlenwasserstofffraktion mit einem reduzierten Benzolgehalt. Bei der Hydrierung von Benzol wird Cyclohexan gebildet. Auch in WO 2010/027987 sind keine Hinweise enthalten, dass aus dem bei der Hydrierung erhaltenen Gemisch Cyclohexan isoliert werden kann, vielmehr soll auch dieses Verfahrensprodukt zur Benzinherstellung verwendet werden. Ebenso wenig offenbart dieses Dokument eine Isomerisierung von MCP zu Cyclohexan oder die Anwesenheit von DMP im Kohlenwasserstoff-Ausgangsgemisch.

US-A 3,311,667 betrifft ein Verfahren zur Entfernung von Benzol aus einem Gemisch, das nachfolgend in eine Isomerisierung von MCP zu Cyclohexan eingespeist wird. Bei der Hydrierung wird Benzol in Gegenwart eines geeigneten Katalysators, beispielsweise einem Metallkatalysator auf Kieselgur, mit Wasserstoff zu Cyclohexan hydriert. Die Isomerisierung von MCP zu Cyclohexan wird in Gegenwart von Metallhalogeniden wie säureverstärktem Aluminiumhalid, durchgeführt. In diesem Dokument sind jedoch keine Angaben enthalten, ob DMP vorhanden ist und somit an welcher Stelle DMP von Cyclohexan abgetrennt wird bzw. dass diese Abtrennung problematisch ist.

EP-A 1 995 297 offenbart ein Verfahren sowie eine zugehörige Vorrichtung zur Hydrierung und Decyclisierung von Benzol und der Isomerisierung von $C_5$-$C_6$-Paraffinen, die in einem Gemisch enthalten sind, das höchstens 1 Gew.-% Benzol enthält. Zur Hydrierung von Benzol können metallhaltige Katalysatoren verwendet werden, wobei sich als Metall die Elemente der Platingruppe, Zinn oder Kobalt und Molybdän eignen. Zur Isomerisierung des bei der Hydrierung erhaltenen Gemisches, das eine Restmenge an Benzol enthalten kann, werden insbesondere Zeolithe als Katalysator eingesetzt. Bei dem in EP-A 1 995 297 beschriebenen Verfahren werden bei der Isomerisierung die Parameter so eingestellt, dass eine Öffnung der bei der Benzolhydrierung erhaltenen Cyclohexanringe zu Isoalkanen erzielt wird. Bei diesem Verfahren geht es also nicht vordergründig um die Herstellung von Cyclohexan, sondern um die Herstellung von Alkanen mit einem hohen Verzweigungsgrad. Darüber hinaus sind auch in EP-A 1 995 297 keine Angaben enthalten, dass zur Isomerisierung auch eine saure ionische Flüssigkeit eingesetzt werden kann bzw. dass die Abtrennung von Aromaten, insbesondere von Benzol, vor der Isomerisierung verteilhaft ist. Ein sinngemäßes Verfahren zu EP-A 1 995 297 wird in EP-A 1 992 673 beschrieben.

US-A 2,846,485 offenbart ein Verfahren zur Herstellung von hochreinem Cyclohexan und Benzol, wobei ein Gemisch eingesetzt wird, das n-Hexan, Benzol, MCP, Cyclohexan und DMP enthält. In einer ersten Extraktivrektifikationszone wird Benzol von den übrigen Eduktkomponenten abgetrennt. Das von Benzol weitgehend befreite Edukt wird mit einem Gemisch vereinigt, das Cyclohexan und MCP enthält und aus dem Sumpf einer zweiten fraktionierenden Rektifikationszone stammt. Das so vereinigte Gemisch wird in eine erste fraktionierende Rektifikationszone eingespeist, wobei über Kopf eine MCP-haltige Fraktion und aus dem Sumpf eine cyclohexanhaltige Fraktion abgetrennt wird.

Das Überkopfprodukt der ersten fraktionierenden Rektifikationszone wird zunächst in eine Isomerisierungszone geführt, in der die Hauptmenge an MCP zu Cyclohexan unter Verwendung von Friedel-Crafts-Katalysatoren wie Aluminiumchlorid, das zusätzlich HCl enthalten kann, isomerisiert wird. Das Isomerisierungsprodukt wird in die vorstehend beschriebene zweite fraktionierende Rektifikationszone eingeleitet, um dort n-Hexan und Leichtsieder als Kopfprodukt abzutrennen. Das Sumpfprodukt aus der ersten fraktionierenden Rektifikationszone wird in eine zweite Extraktivrektifikationszone überführt, in der aus dem Sumpf ein Cyclohexan enthaltendes Gemisch von dem über Kopf abgezogenen DMP abgetrennt wird.

Das in US-A 2,846,485 beschriebene Verfahren ist nachteilig, da es (unter anderem) apparativ sehr aufwändig ist. Die Abtrennung des eigentlichen Verfahrensproduktes Cyclohexan von DMP erfolgt erst zum Schluss des Verfahrens, da das bei der Isomerisierung von MCP gebildete Cyclohexan in eine DMP-haltige Fraktion rückgeleitet wird, d.h. das DMP muss von der gesamten Menge des produzierten Cyclohexans abgetrennt werden. Weiterhin wird in diesem Verfahren zuerst das Benzol abgetrennt, um dieses als eigenständiges Produkt zu gewinnen. Die Benzolabtrennung ist aber apparativ aufwändiger als die Hydrierung von Benzol zu Cyclohexan gemäß dem Verfahren der vorliegenden Erfindung.

US-A 3,406,217 betrifft ein Verfahren zur Herstellung von Cyclohexan aus einer Petroleum-Naphtha-Fraktion, die Benzol, Methylcyclopentan, Cyclohexan und paraffinische Kohlenwasserstoffe mit einem bis acht Kohlenstoffatomen enthält. In Schritt a) wird die (Petroleum-) Naphtha-Fraktion in eine Destillationszone eingeleitet. Gemäß Schritt b) werden in der Destillationszone i) eine Zwischenfraktion, die Benzol, Methylcyclopentan und Cyclohexan umfasst, ii) eine Überkopffraktion, die Pentane und leichtere paraffinische Kohlenwasserstoffe umfasst, sowie iii) eine Sumpffraktion, die Heptane und schwerere paraffinische Kohlenwasserstoffe umfasst, voneinander abgetrennt. Gemäß Schritt c) wird die Sumpffraktion in eine Crackingzone geleitet, wo zumindest ein Teil der Sumpffraktion in Benzol, Olefine und Diolefine umgewandelt wird. In Schritt d) wird ein benzolhaltiger Strom aus dieser Crackingzone entfernt und mit der Zwischenfraktion gemäß Schritt b) vereinigt. Aus diesem vereinten Strom gemäß Schritt d) werden in Schritt e) die Paraffinkomponenten entfernt, um einen "Benzol-Hydrierungseinspeisungsstrom" zu erzeugen. Dieser Strom wird in Schritt f) durch eine Benzolhydrierungszone geleitet, wobei Benzol in Cyclohexan umgewandelt wird. Der Ausfluss aus der Benzolhydrierungszone wird in Schritt g) durch eine Isomerisierungszone geleitet, wo Methylcyclopentan in Cyclohexan umgewandelt wird. Gemäß Schritt h) wird Cyclohexan aus der Isomerisierungszone entfernt. Im Verfahren gemäß US-A 3,406,217 wird bei der Isomerisierung ein konventioneller Isomerisierungskatalysator wie ein mit HCl versehener Aluminiumhalogenid-Komplexkatalysator verwendet. Die Abtrennung des Cyclohexans von DMP spielt bei diesem Verfahren gar keine Rolle, da die in US-A 3,406,217 eingesetzten Ausgangsgemische gar kein DMP enthalten.

US-B 6,503,465 offenbart ein System zur Isomerisierung eines Kohlenwasserstoff-Feedstocks umfassend gesättigte $C_6$-Kohlenwasserstoffe. Das System umfasst einen ersten Isomerisierungsreaktor enthaltend einen ersten Isomerisierungskatalysator sowie insgesamt 14 verschiedene Leitungsvorrichtungen, die in einem sehr komplexen Zusammenhang miteinander stehen. In diesem System sind insgesamt zwei Isomerisierungsreaktoren und drei Separatoren miteinander verschaltet. Die einzige Figur von US-B 6,503,465 veranschaulicht ein solches System. In dem Verfahren spielen auch Ströme eine Rolle, die Cyclohexan, Isohexan, Methylcyclopentan und/oder n-Hexan umfassen können. Cyclohexan ist jedoch kein gewünschtes Zielprodukt, vielmehr steht die Gewinnung von Isohexan sowie von n-Hexan im Vordergrund. Eine Hydrierung von Benzol zu Cyclohexan sowie die Trennproblematik eines Cyclohexan/DMP-Gemisches spielen im Verfahren gemäß US-B 6,503,465 jedoch keine Rolle.

Ionische Flüssigkeiten eignen sich unter anderem als Katalysatoren für die Isomerisierung von Kohlenwasserstoffen. Eine entsprechende Verwendung einer ionischen Flüssigkeit ist beispielsweise in WO 2011/069929 offenbart, wo eine spezielle Auswahl von ionischen Flüssigkeiten in Gegenwart eines Olefins zur Isomerisierung von gesättigten Kohlenwasserstoffen eingesetzt wird, insbesondere zur Isomerisierung von Methylcyclopentan (MCP) zu Cyclohexan. Ein sinngemäßes Verfahren ist in WO 2011/069957 beschrieben, allerdings erfolgt dort die Isomerisierung nicht in Gegenwart eines Olefins, sondern mit einer Kupfer (II)-Verbindung.

Die der vorliegenden Erfindung zugrunde liegende Aufgabe besteht in der Bereitstellung eines neuen Verfahrens zur Herstellung von Cyclohexan aus einem Kohlenwasserstoffgemisch enthaltend Benzol, MCP, DMP und gegebenenfalls mindestens einen Leichtsieder. Weiterhin soll die Möglichkeit bestehen, dass gegebenenfalls im Kohlenwasserstoffgemisch enthaltenes Cyclohexan sowie das bei der Hydrierung von Benzol gebildete Cyclohexan zurückgewonnen werden kann.

Gelöst wird die Aufgabe durch ein Verfahren zur Herstellung von Cyclohexan umfassend die folgenden Schritte

a) Hydrierung eines Kohlenwasserstoffgemisches (KG1), wobei (KG1) i) Benzol, ii) Methylcyclopentan (MCP), iii) Dimethylpentane (DMP), iv) gegebenenfalls Cyclohexan und v) gegebenenfalls mindestens eine Verbindung ausgewählt aus nicht cyclischen $C_5$-Alkanen, Cyclopentan oder nicht cyclischen $C_6$-Alkanen enthält, unter Erhalt eines Kohlenwasserstoffgemisches (KG2), das eine gegenüber (KG1) erhöhte Menge an Cyclohexan aufweist,

b) Einspeisen des Kohlenwasserstoffgemisches (KG2) in eine Rektifikationskolonne (D1),

c) Abtrennen eines Stromes (S1) enthaltend DMP und Cyclohexan aus dem Kohlenwasserstoffgemisch (KG2) über einen Auslass der Rektifikationskolonne (D1), wobei sich der Auslass unterhalb des Zulaufs, bevorzugt am Sumpf von (D1) befindet, unter Erhalt des Kohlenwasserstoffgemisches (KG2a), das eine gegenüber (KG2) reduzierte Menge an DMP aufweist,

d) gegebenenfalls Abtrennen von mindestens einer Verbindung ausgewählt aus nicht cyclischen $C_5$-Alkanen, Cyclopentan oder nicht cyclischen $C_6$-Alkanen aus dem Kohlenwasserstoffgemisch (KG2a) in einer Rektifikationskolonne (D3) unter Erhalt des Kohlenwasserstoffgemisches (KG2b), das eine gegenüber (KG2a) reduzierte Menge an mindestens einer Verbindung ausgewählt aus nicht cyclischen $C_5$-Alkanen, Cyclopentan oder nicht cyclischen $C_6$-Alkanen aufweist,

e) Isomerisierung des Kohlenwasserstoffgemisches (KG2a) oder gegebenenfalls des Kohlenwasserstoffgemisches (KG2b) in Gegenwart eines Katalysators unter Erhalt eines Kohlenwasserstoffgemisches (KG3), das eine gegenüber (KG2a) oder gegebenenfalls gegenüber (KG2b) erhöhte Menge an Cyclohexan aufweist,

f) Isolierung von Cyclohexan aus dem Kohlenwasserstoffgemisch (KG3).

Durch das erfindungsgemäße Verfahren kann in vorteilhafter Weise reines, insbesondere hochreines (spezifikationsgerechtes) Cyclohexan hergestellt werden, wobei die Spezifikationen beispielweise durch die Verwendung des Cyclohexans für die dem Fachmann bekannte Herstellung von Caprolactam gegeben sind. Das erfindungsgemäße Verfahren ist vorteilhaft hinsichtlich des apparativen Aufwandes, weiterhin können hohe Ausbeuten an Cyclohexan erhalten werden.

Aufgrund der (Vor)-Abtrennung von DMP gemäß Schritt c) ("DMP-Vorabtrennung") vor dem zweiten Teil der Cyclohexan-Herstellungsprozess infolge von Isomerisierung gemäß Schritt e) kann die äußerst aufwändige Trennung, insbesondere Rektifikation, von DMP aus dem Verfahrensprodukt Cyclohexan zumindest teilweise umgangen werden, insbesondere wenn es sich bei dem DMP um 2,4-Dimethylpentan (2,4-DMP) handelt und dieses im Ausgangsgemisch in einer Konzentrationen > 100 ppm vorliegt, Dadurch wird der energetische und apparative Aufwand bei der Herstellung von reinem bzw. hochreinem Cyclohexan deutlich reduziert.

Durch das erfindungsgemäße Verfahren kann in vorteilhafter Weise das im Ausgangsgemisch enthaltene DMP vollständig oder nahezu vollständig durch die (Vor)-Abtrennung aus dem Ausgangsgemisch entfernt werden. Besonders bevorzugt wird das erfindungsgemäße Verfahren so durchgeführt, dass das im Ausgangsgemisch enthaltene DMP vollständig oder nahezu vollständig (bis zu 2 % bezogen auf die im Ausgangsgemisch enthaltenen Menge aller DMP-Isomere) per DMP-Abtrennung aus dem Ausgangsgemisch abgetrennt wird. Alternativ kann eine nahezu vollständige DMP-Abtrennung aus dem Ausgangsgemisch auch über die im Gemisch (KG2a) verbliebene DMP-Menge in Bezug auf MCP definiert werden. Bei dieser Betrachtungsweise ist es besonders bevorzugt, dass die in der Rektifikationsvorrichtung (D1) als Gemisch (KG2a) vorzugsweise über Kopf abgezogene DMP-Menge bezogen auf die Summe der über Kopf abgezogenen Mengen an MCP höchstens 0,1 Gew.-%, bevorzugt höchstens 0,02 Gew.-% beträgt.

Das erfindungsgemäße Verfahren kann unabhängig davon durchgeführt werden, ob im eingesetzten Kohlenwasserstoffgemisch (Ausgangsgemisch) bereits Cyclohexan enthalten ist oder nicht. Sofern in den eingesetzten Kohlenwasserstoffgemischen neben DMP auch Cyclohexan selber enthalten ist, wird dieses im Ausgangsgemisch enthaltene Cyclohexan sowie das bei der Benzolhydrierung gemäß Schritt a) gebildete Dyclohexan im Rahmen des erfindungsmäßen Verfahrens zusammen mit DMP bevorzugt über Sumpf abgetrennt. Der mit dieser Fallkonstellation verbundene Nachteil einer Verringerung der Cyclohexan-Produktmenge wird durch die vorstehend beschriebene Verringerung des energetischen und apparativen Aufwands jedoch überkompensiert.

In einer Ausführungsform der vorliegenden Erfindung kann dieses im Kohlenwasserstoff-Ausgangsgemisch enthaltene Cyclohexan jedoch wieder zurückgewonnen werden. Bei dieser Ausführungsform wird das gemeinsam mit dem DMP aus dem Verfahren ausgeschleuste Cyclohexan destillativ, vorzugsweise durch eine Extraktiv- oder Schleppmittelrektifikation, von DMP wieder abgetrennt. Das dabei erhaltene Cyclohexan, das im Wesentlichen frei von DMP ist, kann isoliert und/oder dem Verfahrensprodukt (Cyclohexan, das nach dem erfindungsgemäßen Verfahren in Schritt e) hergestellt wird) wieder zugeführt oder an einer sonstigen Stelle in das erfindungsgemäße Verfahren eingespeist werden. Der Vorteil bei dieser Verfahrensvariante gegenüber einer Abtrennung aus einer Stelle weiter hinten im Verfahren (stromabwärts), also z. B. aus dem Cyclohexan-Produktstrom, ist darin zu sehen, dass die DMP-Abtrennung von einer deutlich kleineren Cyclohexanmenge durchzuführen ist, da DMP nur von dem gegebenenfalls im Kohlenwasserstoff-Ausgangsgemisch enthaltenen Cyclohexan sowie dem bei der Hydrierung gebildeten Cyclohexan abgetrennt wird und nicht auch von dem bei der Isomerisierung gebildeten Cyclohexan, das einen wesentlichen Teil des eigentlichen Verfahrensprodukts darstellt. Demzufolge sind für diese separate DMP/Cyclohexan-Trennung kleinere Apparaturen und eine geringere Energiemenge erforderlich.

Weiterhin kann im erfindungsgemäßen Verfahren aufgrund der gemäß Schritt a) vorgeschalteten Hydrierung der Aromaten, insbesondere von Benzol, die Isomerisierung gemäß Schritt e) in vorteilhafter Weise durchgeführt werden. Der Vorteil ist darin zu sehen, dass die in (KG1) enthaltenen Aromaten, insbesondere Benzol, durch eine vorgeschaltete Hydrierung vollständig oder zumindest weitgehend abgetrennt in die entsprechenden gesättigten Kohlenwasserstoffe umgewandelt werden können. Demzufolge wird die andernfalls eintretende Desaktivierung der zur Isomerisierung, insbesondere zur Isomerisierung von MCP zu Cyclohexan, eingesetzten Katalysatoren durch Aromaten, insbesondere durch Benzol oder andere ungesättigte Verbindungen, die vor allem bei der bevorzugten Verwendung von sauren ionischen Flüssigkeiten als Katalysatoren zum Tragen kommt, verringert oder ganz vermieden.

Weiterhin hat die Hydrierung des in (KG1) enthaltenen Benzols den Vorteil, dass die Menge des anfallenden Produkts um das bei der Hydrierung des Benzols anfallende Cyclohexan erhöht wird, sofern das bei der Hydrierung anfallende Cyclohexan, das erfindungsgemäß vollständig oder zumindest größtenteils zusammen mit DMP gemäß Schritt c) aus dem Verfahren ausgeschleust wird, wieder mit der vorstehend beschriebenen DMP/Cyclohexan-Trennung rückgewonnen werden.

Die Entfernung der verbliebenen Aromaten, insbesondere von Benzol, mittels Hydrierung hat darüber hinaus den zusätzlichen Vorteil, dass die nachfolgend ausgeführten destillativen Aufarbeitungsschritte, insbesondere gemäß optimalem Schritt d), erleichtert werden, weil so die andernfalls auftretende Bildung von Azeotropen von Aromaten wie zum Beispiel Benzol mit gesättigten $C_6$-$C_7$-Alkanen vermieden wird.

Prinzipiell kann eine optionale Abtrennung von Leichtsiedern, also eines Großteils der im Kohlenwasserstoffgemisch (KG1) optional enthaltenen nicht-cyclischen $C_5$-$C_6$-Alkane sowie Cyclopentan, insbesondere von Isohexanen, an verschiedenen Stellen im Verfahren erfolgen. Besonders vorteilhaft ist es jedoch, im Fall von benzolhaltigen Kohlenwasserstoffgemischen (KG1) die Abtrennung von Leichtsiedern nach der Hydrierung und vor der Isomerisierung durchzuführen. Eine Abtrennung von Leichtsiedern vor der Hydrierung hätte nämlich den Nachteil, dass das vor der Hydrierung im Kohlenwasserstoffgemisch enthaltene Benzol mit mindestens einem Teil der abzutrennenden Leichtsiedern Azeotrope bildet und daher zumindest teilweise zusammen mit den Leichtsiedern abgetrennt werden würde. Dadurch würde sich die Produktmenge um die zusammen mit den Leichtsiedern abgetrennte Menge an Benzol verringern.

Eine Abtrennung von Leichtsiedern nach der Isomerisierung hätte wiederum den Nachteil, dass die Leichtsieder die zu isomerisierenden Kohlenwasserstoffe, insbesondere MCP, verdünnen und so zu einer Verringerung der Raum-Zeit-Ausbeute in der Isomerisierung führen würden. Zudem ist die Abtrennung von Isohexanen vor der Isomerisierung vorteilhaft, weil so die Triebkraft für die Isomerisierung von n-Hexan zu Isohexanen in der nachfolgenden Isomerisierungsstufe erhöht wird. Die Isomerisierung von n-Hexan zu Isohexanen in der Isomerisierungsstufe ist wiederum bedeutsam, weil aufgrund der Lage der Siedepunkte n-Hexan (Normalsiedepunkt 68,7 °C) deutlich schwieriger als die Isohexane (Normalsiedepunkte 49,7 bis 63,3 °C) von MCP (Normalsiedepunkt 71,7 °C) abzutrennen ist. Da sich aber bevorzugt an die Isomerisierungsstufe eine destillative Trennung anschließt, in der MCP zusammen mit offenkettigen Hexanen vom gebildeten Cyclohexan abgetrennt und vor bzw. in die Isomerisierung zurückgeführt werden, was wiederum die Ausschleusung der offenkettigen Hexane aus dem Verfahren nötig macht, ist es aufgrund der genannten Lage der Siedepunkte vorteilhaft, die offenkettigen Hexane überwiegend in Form von Isohexanen aus dem Verfahren auszuschleusen, während eine durch die Isomerisierung von n-Hexan begrenzte Aufpegelung desselben in Kauf genommen werden kann.

Auch im Vergleich zu Verfahren, bei denen zunächst eine Schwersiedervorabtrennung und dann erst eine Benzolhydrierung durchgeführt wird, liefert das erfindungsgemäße Verfahren Vorteile. Insbesondere wird durch die der DMP/Schwersiederabtrennung gemäß Schritt c) erfindungsgemäß vorgeschaltete Benzolhydrierung gemäß Schritt a) die Bildung von Azeotropen von Aromaten, insbesondere Benzol mit gesättigten $C_6$-$C_7$-Alkanen in den darauffolgenden Verfahrensschritten vermieden. Insbesondere liegen im erfindungsgemäßen Schritt c) keine oder nur noch mengenmäßig sehr geringe Benzol-Azeotrope vor. Sämtliche Folgeschritte des Verfahrens arbeiten somit vollständig oder zumindest weitgehend aromatenfrei, was sicherheitstechnische Vorteile bietet, da aromatische Verbindungen in der Regel als stark toxisch eingestuft sind (CMR-Stoff).

In diesem Zusammenhang ist hinsichtlich des Stromes (D1) gemäß Schritt c) die Abzugsmenge, vorzugsweise über den Sumpf, von (D1) zwar um den im eingesetzten Kohlenwasserstoffgemisch enthaltenen Anteil an Benzol, der zuvor zu Cyclohexan hydriert wurde, erhöht. Dadurch erhöht sich weiterhin der Durchsatz in der weiteren Aufarbeitung dieses Stroms, was zu höherem Energieaufwand und größeren Apparaten in der Extraktivdestillation (D2) führt. Dies wird jedoch durch eine Verringerung des Durchsatzes der optionalen Leichtsiederabtrennung und vor allem bei Isomerisierung kompensiert.

Der Strom, der der optionalen Leichtsiederabtrennung zugeführt wird, verringert sich um die aus Benzol gebildete Menge an Cyclohexan. Er enthält in der Regel im Wesentlichen MCP, leichter siedenden Komponenten und etwas Cyclohexan, welches aus der Produktdestillation gegebenenfalls zurückgeführt wird. Die geringere Menge führt zu einer Verminderung des Energieaufwands und kleineren Apparatedimensionen im optionalen Schritt d). Damit verringern sich ebenfalls der Durchsatz und damit die Apparatedimensionen der Isomerisierung gemäß Schritt e). Durch den geringeren Cyclohexangehalt in der Isomerisierung zugeführten Strom wird der Reaktionsumsatz vorteilhaft beeinflusst, da es sich bei einer durch eine ionische Flüssigkeit katalysierten Isomerisierung von MCP zu Cyclohexan um eine Gleichgewichtsreaktion handelt. Infolgedessen können auch in Schritt e) die Vorrichtungen/Reaktoren kleiner dimensioniert werden.

Die destillative Trennung des Cyclohexan von leichter siedenden Komponenten gemäß Schritt f) ist durch die Verfahrensänderung ebenfalls weniger aufwändig, wodurch Energie eingespart werden kann. Außerdem wird der Destillatstrom, welcher gegebenenfalls zur Leichtsiederabtrennung zurückgeführt wird, kleiner.

In Summe können durch die Verfahrensänderung (Hydrierung vor DMP-Abtrennung) der Energiebedarf eingespart sowie die Investkosten verringert werden.

Im Rahmen der vorliegenden Erfindung kann eine Rektifikation in den dem Fachmann bekannten Ausführungsformen (siehe z B. Kirk-Othmer Encyclopedia of Chemical Technology, Published Online: 17 AUG 2001, Vol. 8 S. 739 ff.) durchgeführt werden. Die jeweiligen Rektifikationstechniken werden in den entsprechenden dem Fachmann bekannten Vorrichtungen durchgeführt. Die Durchführung einer Extraktivrektifikation zur Auftrennung engsiedender Substanzen ist beispielsweise in US A 4,053,369, US-A 4,955,468 oder WO 02/22528 beschrieben. Die Rektifikation unter Verwendung von Trennwandkolonnen ist beispielweise in EP1127601 B1 beschrieben.

Unter "Rektifikation", die in einer entsprechenden Rektifizierkolonne (Rektifiziervorrichtung), auch Rektifikationskolonne oder Rektifikationsvorrichtung genannt, durchgeführt wird, wird Folgendes verstanden: Bei der Rektifikation wird der durch Rektifikation erzeugte Dampf in einer Rektifizierkolonne im Gegenstrom zu einem Teil seines Kondensates geführt. Auf diese Weise werden leichter flüchtige Komponenten im Kopf- und schwerer flüchtige im Sumpfprodukt der Rektifizierkolonne angereichert.

Im vorliegenden Zusammenhang schließt der Begriff "Rektifikationskolonne" jeweils dem Fachmann bekannte Nebenapparate wie z. B. einen oder mehrere Sumpfverdampfer, mindestens einen Kondensator sowie gegebenenfalls Behälter und Pumpen, mit ein. Dementsprechend ist die Entnahme von Strömen aus der Rektifikationskolonne so zu verstehen, dass der jeweilige Strom gegebenenfalls über einen oder mehrere dieser Nebenapparate geleitet wird, gegebenenfalls auch unter Änderung des Aggregatzustands und/oder Rückleitung eines Teils des entnommenen Stroms. So ist zum Beispiel die Entnahme eines Stroms über den Kopf der Rektifikationskolonne so zu verstehen, dass der am Kopf der Kolonne anfallende Brüdenstrom mindestens teilweise kondensiert wird und nachfolgend in einen Rücklaufstrom und einen Kopfproduktstrom aufgeteilt wird. Der Kopfproduktstrom ist dann gleichzusetzen mit dem im nachfolgenden Text vereinfachend als über Kopf entnommenen Strom bezeichneten Strom. Analog schließt auch die Nennung der Zuführung eines Stromes zu einer Rektifikationskolonne die Option ein, dass der betreffende Strom vor dem Eintritt in die Kolonne selbst einen oder mehrere Nebenapparate durchläuft wie beispielweise einen Vorwärmer oder Vorverdampfer.

Im Rahmen der vorliegenden Erfindung werden unter dem Begriff "Dimethylpentane" (DMP) alle bekannten Isomere von Dimethylpentan verstanden, insbesondere 2,2-Dimethylpentan (2,2-DMP; Normalsiedepunkt: 79,17 °C), 2,3-Dimethylpentan (2,3-DMP; Normalsiedepunkt: 89,88 °C), 3,3-Dimethylpentan (3,3-DMP; Normalsiedepunkt: 86,09 °C) und 2,4-Dimethylpentan (2,4-DMP; Normalsiedepunkt: 80,52 °C). Dies bedeutet, dass in den entsprechenden Gemischen bzw. Strömen des erfindungsgemäßen Verfahrens mindestens ein Dimethylpentan-Isomer enthalten ist, vorzugsweise handelt es sich um Gemische von zwei oder mehr Dimethylpentan-Isomeren, wobei eines dieser Isomere vorzugsweise 2,4-Dimethylpentan ist.

Im Rahmen der vorliegenden Erfindung werden unter dem Begriff "Verbindungen mit einem Normalsiedepunkt von 79 bis 84 °C" alle Kohlenwasserstoffe verstanden, die bei Normaldruck im Bereich von 79 bis 84 °C sieden und die einzeln oder als Gemisch im erfindungsgemäßen Verfahren zunächst im Kohlenwasserstoffgemisch (KG1) enthalten sein können. Im Rahmen des erfindungsgemäßen Verfahrens können einzelne oder mehrere dieser Verbindungen voneinander abgetrennt werden. Gegebenenfalls können auch einzelne oder mehrere dieser Verbindungen im nachfolgenden Text separat als Bestandteil von Gemischen oder Strömen aufgeführt sein. Sofern dies der Fall ist, sind nur die jeweils konkret aufgeführten Verbindungen zwingender Bestandteil des entsprechenden Gemisches oder Stromes; die sonstigen Verbindungen mit einem Normalsiedepunkt von 79 bis 84 °C, die nicht namentlich im entsprechenden Strom oder Gemisch aufgeführt sind, können (sofern nicht anders aufgeführt oder beispielsweise infolge einer vorausgegangenen Abtrennung nicht mehr möglich) ebenfalls im entsprechenden Strom oder Gemisch vorhanden sein. Gegebenenfalls können einzelne oder mehrere dieser Verbindungen auch unter die Definition einer anderen Auswahl von Verbindungen fallen, wie beispielsweise unter die Definition des Begriffs "$C_5$-$C_6$-Alkane".

Beispiele für Verbindungen mit einem Normalsiedepunkt von 79 bis 84 °C sind Cyclohexan (80,78 °C), 2,2-DMP (79,17 °C), 2,4-DMP (80,52 °C), 2,2,3-Trimethylbutan (80,87 °C) und Benzol (80,08 °C).

Sinngemäßes wie vorstehend für die Verbindungen mit einem Normalsiedepunkt von 79 bis 84 °C aufgeführt gilt im Rahmen der vorliegenden Erfindung auch für Verbindungen, die unter den Begriff "Schwersieder mit einem Normalsiedepunkt > 84 °C" fallen. Beispiele für Schwersieder mit einem Normalsiedepunkt > 84 °C sind 3,3-DMP (86,09 °C), 2,3-DMP (89,88 °C), 2-Methylhexan (2-MH; 90,06 °C), 3-Methylhexan (3-MH; 91,87 °C) und 3-Ethylpentan (3-EP; 93,45 °C).

Im Rahmen der vorliegenden Erfindung können die beiden vorgenannten Gruppen von Verbindungen (Verbindungen mit einem Normalsiedepunkt von 79 bis 84 °C sowie Schwersieder mit einem Normalsiedepunkt > 84 °C) gegebenenfalls auch zu einer Gruppe von Verbindungen zusammengefasst sein. In dieser Fallkonstellation werden die Verbindungen entsprechend als "Schwersieder mit einem Normalsiedepunkt > 78 °C" bezeichnet. Die vorstehenden Ausführungen zu den beiden Einzelgruppen gelten sinngemäß auch für diese Gruppe von Verbindungen.

Weiterhin kann im Rahmen der vorliegenden Erfindung die Gruppe der Verbindungen mit einem Normalsiedepunkt > 84 °C auch als Untergruppe in der Gruppe enthalten sein, die als "schwerer als Cyclohexan siedende Komponenten" bezeichnet wird. Die letztgenannte Gruppe umfasst also zusätzlich auch Verbindungen mit einem Normalsiedepunkt > 80,78 °C bis einschließlich 84 °C.

Im Rahmen der vorliegenden Erfindung bedeutet der Begriff "Großteil" im Zusammenhang mit einem Strom (Feedstrom) -soweit nicht anders ausgeführtmindestens 50 %, vorzugsweise mindestens 80 %, mehr bevorzugt mindestens 95 %, insbesondere mindestens 99 Gew%.

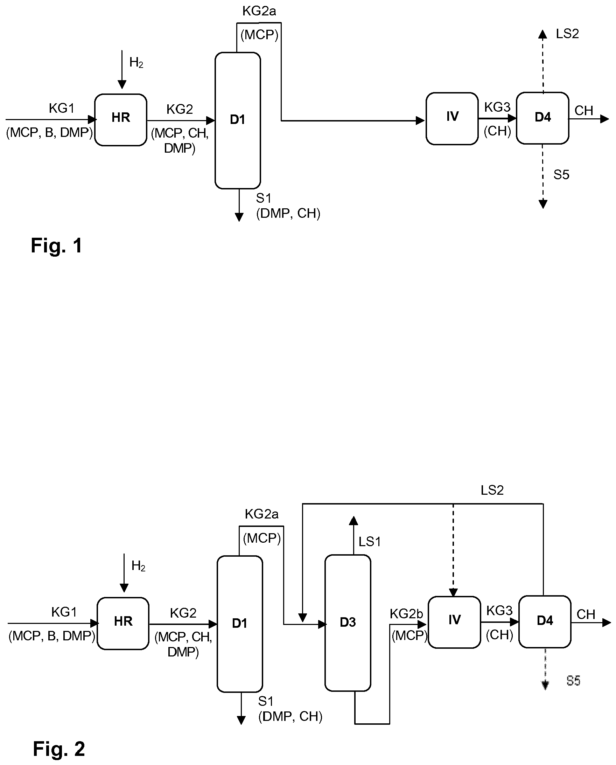

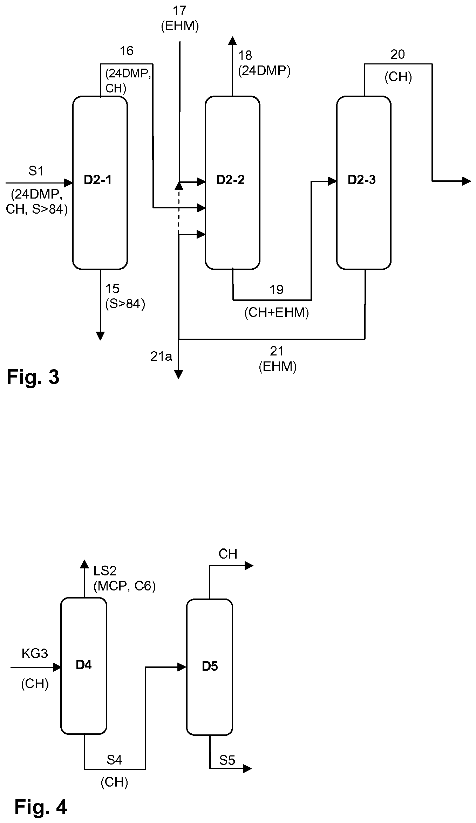

Nachfolgend wird das erfindungsgemäße Verfahren zur Herstellung von Cyclohexan aus Methylcyclopentan (MCP) und Benzol näher definiert. In diesem Zusammenhang wird auch auf die Figuren 1 bis 4 verwiesen. Figur 1 zeigt das erfindungsgemäße Verfahren in seiner Grundform, wobei bei der Isolierung von Cyclohexan gemäß Schritt f) gegebenenfalls vorhandene Leichtsieder und/oder Schwersieder in einer Vorrichtung (D4) von Cyclohexan abgetrennt werden. Die Abtrennung von Cyclohexan aus (D4) ist in Figur 1 nur schematisch dargestellt (beispielsweise erfolgt dies gemäß Figur 4). Figur 2 zeigt eine bevorzugte Ausführungsform des erfindungsgemäßen Verfahrens mit zwischengeschalteter Leichtsiederabtrennung gemäß Schritt d) sowie unter Berücksichtigung von zwei Varianten der Rückleitung von nicht isomerisiertem MCP gemäß Schritt f). Figur 3 zeigt eine spezielle Ausgestaltung zur Rückgewinnung von Cyclohexan, das bei der Hydrierung von Benzol gebildet wird sowie gegebenenfalls zusätzlich zusammen mit DMP bereits im Kohlenwasserstoffgemisch (KG1) enthalten ist. Die Figur 4 betrifft eine spezielle Ausgestaltung der Isolierung von Cyclohexan gemäß Schritt f). Sämtliche Figuren werden im nachfolgenden Text an entsprechender Stelle näher erläutert.

Im Rahmen der vorliegenden Erfindung erfolgt in Schritt a) die Hydrierung eines Kohlenwasserstoffmisches (KG1), wobei (KG1) i) Benzol, ii) Methylcyclopentan (MCP), iii) Dimethylpentane (DMP), iv) gegebenenfalls Cyclohexan und v) gegebenenfalls mindestens eine Verbindung ausgewählt aus nicht cyclischen $C_5$-Alkanen, Cyclopentan oder nicht cyclischen $C_6$-Alkanen enthält, unter Erhalt eines Kohlenwasserstoffgemisches (KG2), das eine gegenüber (KG1) erhöhte Menge an Cyclohexan aufweist.

Die einzelnen Komponenten des Kohlenwasserstoffgemischs (KG1) können in beliebigen Konzentrationen/Verhältnissen untereinander vorliegen. Vorzugsweise enthält das Kohlenwasserstoffgemisch (KG1) zu mindestens 90 Gew.-%, bevorzugt mindestens 95 Gew.-% Kohlenwasserstoffe mit 5 bis 8 Kohlenstoffatomen, unter der Voraussetzung, dass die Kohlenwasserstoffe mit 5 bis 8 Kohlenstoffatomen MCP, Benzol, DMP, gegebenenfalls Cyclohexan und gegebenenfalls mindestens einen Leichtsieder gemäß der vorstehenden Komponente v) umfassen. Weiterhin ist es bevorzugt, dass das Kohlenwasserstoffgemisch (KG1) Cyclohexan enthält, vorzugsweise zu maximal 15 Gew.-%. Die Kohlenwasserstoffe können ansonsten gesättigt oder ungesättigt und/oder zyklisch, linear oder verzweigt sein. Insbesondere enthält das Kohlenwasserstoffgemisch (KG1) zwischen 10 Gew.-% und 60 Gew. %, mehr bevorzugt zwischen 20 Gew.-% und 50 Gew.-%, MCP und/oder zwischen 1 Gew. % und 30 Gew.-%, mehr bevorzugt zwischen 4 Gew.-% und 20 Gew.-% Benzol.

In einer bevorzugten Ausführungsform der vorliegenden Erfindung enthält das Kohlenwasserstoffgemisch (KG1) Benzol, Methylcyclopentan (MCP), DMP, Cyclohexan und mindestens eine Verbindung ausgewählt aus nicht cyclischen $C_5$-Alkanen, Cyclopentan oder nicht cyclischen $C_6$-Alkanen. Gegebenenfalls können in (KG1) mindestens eine weitere Verbindung ausgewählt aus Olefinen oder $C_7$-$C_8$-Alkanen enthalten sein. Der Begriff "Olefin" umfasst neben linearen, einfach ungesättigten Olefinen wie Penten oder Hexen auch cyclische Olefine, insbesondere Cyclohexen, sowie Diene und cyclische Diene. In der Gruppe der $C_7$-$C_8$-Alkane sind vorzugsweise Verbindungen mit einem Normalsiedepunkt > 78 °C enthalten, im Folgenden auch "Schwersieder" genannt. Gegebenenfalls können im Kohlenwasserstoffgemisch (KG1) auch Kohlenwasserstoffe enthalten sein, die mehr als acht Kohlenstoffatome aufweisen und/oder Kohlenwasserstoffe mit einem relativ niedrigen Siedepunkt, beispielsweise solche, die weniger als fünf Kohlenstoffatome aufweisen. Sinngemäßes gilt auch für die Anwesenheit von weiteren Aromaten neben Benzol.

Besonders bevorzugt enthält das Kohlenwasserstoffgemisch (KG1) Benzol, Methylcyclopentan (MCP), DMP, Cyclohexan, mindestens einen weiteren Kohlenwasserstoff ausgewählt aus n-Hexan und iso-Hexanen und gegebenenfalls mindestens einen weiteren Kohlenwasserstoff ausgewählt aus n-Heptan, iso-Heptanen, Methylcyclohexan oder Dimethylcyclopentanen.

Aufgrund von Schritt a) wird im erfindungsgemäßen Verfahren also Benzol zu Cyclohexan hydriert. In anderen Worten ausgedrückt bedeutet dies, dass in Schritt a) die im Kohlenwasserstoffgemisch (KG1) enthaltenen Aromaten, also Benzol sowie gegebenenfalls enthaltene sonstige Aromaten, unter Erhalt der entsprechenden nichtaromatischen Kohlenwasserstoffe, vorzugsweise der sich unter Erhalt aller Kohlenstoff-Kohlenstoff-Bindungen ergebenden vollständig gesättigten Kohlenwasserstoffe, hydriert werden. Sofern im Kohlenwasserstoffgemisch (KG1) sonstige ungesättigte Verbindungen enthalten sind, beispielsweise Olefine wie Cyclohexen, werden diese in Schritt a) der vorliegenden Erfindung ebenfalls hydriert.

Die Hydrierung des Kohlenwasserstoffgemischs (KG1) gemäß Schritt a) erfolgt im Rahmen der vorliegenden Erfindung in einer hierfür geeigneten Vorrichtung (V), die vorzugsweise mindestens einen Hydrierreaktor (HR) umfasst. In der Vorrichtung (V) wird Benzol zu Cyclohexan hydriert, wobei die Hydrierung vorzugsweise unter Verwendung von elementarem Wasserstoff erfolgt. Weiterhin ist es bevorzugt, dass die Hydrierung in flüssiger Phase erfolgt.

Die Hydrierung von Benzol zu Cyclohexan gemäß Schritt a) wird in der Regel in Gegenwart eines geeigneten Katalysators durchgeführt. Als Katalysatoren eignen sich prinzipiell alle dem Fachmann hierfür bekannten Katalysatoren, beispielsweise ein Metallkatalysator auf Kieselgur gemäß US-A 3,311,667 oder metallhaltige Katalysatoren gemäß EP A 1 995 297, wobei dort als Metall die Elemente der Platingruppe, Zinn oder Kobalt und Molybdän bevorzugt verwendet werden.

Vorzugsweise wird die Hydrierung in Gegenwart eines Katalysators durchgeführt, der als Aktivmetall (auch als Metallkomponente beziehungsweise Aktivkomponente bezeichnet) mindestens ein Element der 8. bis 10. Gruppe des Periodensystems der Elemente (PSE), beispielsweise Eisen, Kobalt, Nickel oder Ruthenium (entspricht der Nebengruppe VIIIB der CAS-Version des PSE) enthält, insbesondere Nickel oder Ruthenium. Weiterhin ist es bevorzugt, dass das Aktivmetall auf einem Trägermaterial (Träger) aufgebracht ist. Als Träger eignen sich prinzipiell alle dem Fachmann bekannten Träger, beispielsweise $SiO_2$-haltige, zirconiumoxidhaltige oder aluminiumoxidhaltige Träger. Besonders bevorzugt wird ein Katalysator eingesetzt, der Nickel als Aktivmetall auf einem aluminiumoxidhaltigen Träger enthält.

Die Hydrierung als solche wird in der dem Fachmann an sich bekannten Art ausgeführt und betrieben, bevorzugt ist eine Kombination aus einem im gegebenenfalls gekühlten Kreislauf betriebenen Hauptreaktor (Rückführung eines Teils des aus dem Reaktor ausströmenden Gemischs in das dem Reaktor zuströmende Gemisch, wobei gegebenenfalls die Kühlung vor oder nach der genannten Zuführung platziert ist) und einem nachfolgenden im geraden Durchgang, das heißt ohne Rückführung betriebenen Nachreaktor. In diesem Fall umfasst die Vorrichtung (V) also zwei Hydrierreaktoren (HR).

Die Hydrierreaktoren (HR) sind bevorzugt als Festbettreaktoren ohne interne Kühlung ausgelegt. In diesem Fall wird die Hydrierung bevorzugt so betrieben, dass die Temperaturdifferenz zwischen eintretendem und austretendem Gemisch kontinuierlich überwacht und bei Absinken dieses Werts unter einen bestimmten Sollwert die Eintrittstemperatur angehoben wird. Weiterhin ist bevorzugt, dass die Hydrierreaktoren in Rieselfahrweise betrieben werden.

Weiterhin bevorzugt wird der Hydrierung ein Apparat nachgeschaltet, in dem auf einen Druck unterhalb des im Nachreaktor eingestellten Drucks entspannt wird. Dabei fällt ein Gasstrom an, der zuvor im Kohlenwasserstoffgemisch eingelösten Wasserstoff enthält und jedenfalls verdichtet und in mindestens einen der Hydrierreaktoren (HR) zurückgeführt wird.

Die Hydrierung wird bevorzugt bei einer Temperatur zwischen 50 und 200 °C, besonders bevorzugt zwischen 100 und 180 °C und/oder einem Druck zwischen 10 und 300 bar abs., besonders bevorzugt zwischen 30 und 200 bar abs. durchgeführt.

Weiterhin ist es im erfindungsgemäßen Verfahrens bei der Hydrierung bevorzugt, dass der Gesamtumsatz des Benzols (und gegebenenfalls sonstiger im Kohlenwasserstoffgemisch (KG1) enthaltener ungesättigter Verbindungen) mindestens 90 %, besonders bevorzugt 99 % beträgt und/oder der Restgehalt des Benzols (und gegebenenfalls sonstiger im Kohlenwasserstoffgemisch (KG1) enthaltenen ungesättigten Verbindungen) im Kohlenwasserstoffgemisch (KG2) 1 Gew.-%, bevorzugt höchstens 0,1 Gew.-%, besonders bevorzugt höchstens 0,01 Gew.-% beträgt.

Infolge der Hydrierung wird im erfindungsgemäßen Schritt a) das Kohlenwasserstoffgemisch (KG2) erhalten, das sich in seiner Zusammensetzung vom Kohlenwasserstoffgemisch (KG1) vordergründig hinsichtlich der hydrierten Verbindungen unterscheidet. Das Kohlenwasserstoffgemisch (KG2) enthält also DMP, Cyclohexan, MCP und mindestens eine Verbindung ausgewählt aus nicht cyclischen $C_5$-Alkanen, Cyclopentan oder nicht cyclischen $C_6$-Alkanen (Leichtsieder). Im Kohlenwasserstoffgemisch (KG2) sind somit auch die Komponenten ii), iii) und v) vorhanden, die bereits in (KG1) enthalten gewesen sind. Weiterhin enthält das Kohlenwasserstoffgemisch (KG2) alle sonstigen Komponenten gemäß dem Kohlenwasserstoffgemisch (KG1), die bei der Hydrierung chemisch nicht geändert werden, sowie gegebenenfalls durch Hydrierung von Olefinen, Dienen sowie von sonstigen Aromaten gebildeten Kohlenwasserstoffe.

Das Kohlenwasserstoffgemisch (KG2) enthält vorzugsweise Cyclohexan, MCP, DMP, maximal 0,1 Gew.-% an Aromaten und mindestens eine Verbindung ausgewählt aus nicht cyclischen $C_5$-Alkanen, Cyclopentan oder nicht cyclischen $C_6$-Alkanen. Besonders bevorzugt enthält das Kohlenwasserstoffgemisch (KG2) Cyclohexan, Methylcyclopentan (MCP), DMP, maximal 0,1 Gew.-% an Aromaten und mindestens einen weiteren Kohlenwasserstoff ausgewählt aus n-Hexan und iso-Hexanen.

In Schritt b) des erfindungsgemäßen Verfahrens erfolgt das Einspeisen des (bei der Hydrierung gemäß Schritt a)) erhaltenen Kohlenwasserstoffgemisches (KG2) in die Rektifikationskolonne (D1), die nachfolgend im Zusammenhang mit Schritt c) weiter spezifiziert wird.

In Schritt c) des erfindungsgemäßen Verfahrens erfolgt das Abtrennen eines Stromes (S1) enthaltend DMP und Cyclohexan aus dem Kohlenwasserstoffgemisch (KG2) über einen Auslass der Rektifikationskolonne (D1), wobei sich der Auslass unterhalb des Zulaufs, bevorzugt am Sumpf von (D1) befindet, unter Erhalt des Kohlenwasserstoffgemisches (KG2a), das eine gegenüber (KG2) reduzierte Menge an DMP aufweist. Das Kohlenwasserstoffgemisch (KG2a) wiederum wird über einen Auslass der Rektifikationskolonne (D1) oberhalb des Zulaufs, bevorzugt über den Kopf von (D1), abgezogen.

Vorzugsweise wird in der Rektifikationskolonne (D1) das im Kohlenwasserstoffgemisch (KG2) enthaltene DMP und Cyclohexan sowie gegebenenfalls sonstige Alkane mit 7 oder mehr Kohlenstoffatomen (Schwersieder) vollständig oder nahezu vollständig (bis zu 2 % bezogen auf die in (KG2) enthaltene DMP- und Cyclohexan- bzw. Schwersieder-Menge) aus (KG2), insbesondere von MCP und gegebenenfalls den Leichtsiedern gemäß Komponente v) (also den Hauptkomponenten des Gemisches (KG2a)) abgetrennt. Das DMP und Cyclohexan sowie gegebenenfalls sonstige Alkane mit 7 oder mehr Kohlenstoffatomen werden aus der Rektifikationskolonne (D1) als Strom (S1) abgezogen, der sich vorzugsweise im Sumpf von (D1) befindet. Das im Strom (S1) ebenfalls enthaltene Cyclohexan umfasst das Cyclohexan, das im vorausgegangenen Hydrierungsschritt a) aus Benzol gebildet wurde sowie das Cyclohexan, das gegebenenfalls bereits im Kohlenwasserstoffgemisch (KG1) enthalten gewesen ist. Im Kohlenwasserstoffgemisch (KG2a) ist hingegen vorzugsweise kein Cyclohexan oder nur ein relativ geringer Anteil (Maximal 2 Gew.-% bezogen auf (KG2)) enthalten.

Alternativ kann eine nahezu vollständige Schwersieder-Abtrennung, vorzugsweise eine nahezu vollständige DMP-Abtrennung, aus dem Kohlenwasserstoffgemisch (KG2) auch über die im Gemisch (KG2a) verbliebene Schwersieder-Menge, vorzugsweise DMP-Menge, in Bezug auf MCP definiert werden. Bei dieser Betrachtungsweise ist es besonders bevorzugt, dass die im Gemisch (KG2a) enthaltene Schwersieder-Menge, vorzugsweise DMP-Menge, bezogen auf die Summe der in (KG2a) enthaltenen Mengen an MCP höchstens 0,1 Gew.-%, bevorzugt höchstens 0,02 Gew.-% beträgt.

Weiterhin ist es bevorzugt, dass das Kohlenwasserstoffgemisch (KG2a) mindestens 95 %, vorzugsweise mindestens 98 % der im Kohlenwasserstoffgemisch (KG2) enthaltenen Teilmenge an MCP enthält und/oder dass das Kohlenwasserstoffgemisch (KG2a) höchstens 0,1 Gew.-%, vorzugsweise höchstens 0,02 Gew.-% (bezogen auf die Gesamtmenge an MCP in (KG2a)) DMP enthält. Besonders bevorzugt enthält das Kohlenwasserstoffgemisch (KG2a) höchstens 0,015 Gew.-% (bezogen auf die Gesamtmenge an MCP in (KG2a)) 2,4-DMP.

Als Rektifikationskolonne (D1) -gemäß der erfindungsgemäßen Schritte b) und c)-können prinzipiell alle dem Fachmann bekannten Rektifikationskolonne verwendet werden. Weiterhin ist es bevorzugt, dass sich der Auslass der Rektifikationskolonne (D1), aus dem das Gemisch (KG2a) abgetrennt wird, oberhalb des Zulaufs befindet, mit dem das Kohlenwasserstoffgemisch (KG2) in (D1) eingespeist wird, vorzugsweise befindet sich der Auslass im Kopf von (D1).

Der aus dem Sumpf der Rektifikationskolonne (D1) abgetrennte Strom (S1) enthält vorzugsweise DMP, Cyclohexan sowie gegebenenfalls weitere Komponenten. Die weiteren Komponenten sind vorzugsweise Schwersieder mit einem Normalsiedepunkt > 78 °C.

Im optionalen Schritt d) des erfindungsgemäßen Verfahrens erfolgt das Abtrennen von mindestens einer Verbindung ausgewählt aus nicht cyclischen $C_5$-Alkanen, Cyclopentan oder nicht cyclischen $C_6$-Alkanen aus dem Kohlenwasserstoffgemisch (KG2a) in einer Rektifikationskolonne (D3) unter Erhalt des Kohlenwasserstoffgemisches (KG2b), das eine gegenüber (KG2a) reduzierte Menge an mindestens einer Verbindung ausgewählt aus nicht cyclischen $C_5$-Alkanen, Cyclopentan oder nicht cyclischen $C_6$-Alkanen aufweist. Der optionale Schritt d) wird im erfindungsgemäßen Verfahren vorzugsweise dann durchgeführt, wenn im bei der Hydrierung gemäß Schritt a) eingesetzten Kohlenwasserstoffgemisch (KG1) die Komponente v) enthalten ist.

Diese Abtrennung gemäß Schritt d) wird nachfolgend auch als "Leichtsiederabtrennung" bezeichnet. Unter "Leichtsieder" versteht man insbesondere Cyclopentan sowie nicht-cyclische $C_5$-$C_6$-Alkane wie iso-Hexane.

Das um die Leichtsieder abgereicherte Kohlenwasserstoffgemisch (KG2b) wird anschließend der Isomerisierung gemäß Schritt e) der vorliegenden Erfindung zugeführt. Sofern keine Leichtsiederabtrennung gemäß optionalem Schritt d) erfolgt, wird statt (KG2b) das Kohlenwasserstoffgemisch (KG2a) der Isomerisierung gemäß Schritt e) zugeführt. Das um die Leichtsieder abgereicherte Kohlenwasserstoffgemisch (KG2b) wird über eine Abnahmestelle unterhalb des Zulaufs, vorzugsweise aus dem Sumpf der entsprechenden Rektifikationskolonne abgetrennt.

Vorzugsweise wird die Leichtsiederabtrennung so durchgeführt, dass aus dem Kohlenwasserstoffgemisch (KG2a) ein Strom (LS1) enthaltend mindestens eine Verbindung ausgewählt aus linearen oder verzweigten $C_5$-Alkanen, Cyclopentan oder linearen oder verzweigten $C_6$-Alkanen, besonders bevorzugt iso-Hexane, destillativ abgetrennt wird. Vorzugsweise wird der Strom (LS1) über eine Abnahmestelle oberhalb des Zulaufs, besonders bevorzugt über den Kopf der Rektifikationskolonne abgezogen.

In der Rektifikationskolonne (D3) werden die Leichtsieder aus dem Kohlenwasserstoffgemisch (KG2a) als Strom (LS1) abgetrennt, wobei der Strom (LS1) niedriger siedet als (KG2a). Der Strom (LS1) ist gegenüber (KG2a) vorzugsweise an Isohexanen und/oder Cyclopentan angereichert und an MCP und gegebenenfalls vorhandenen Cyclohexan abgereichert. Das um den Strom (LS1) abgereicherte/verminderte Kohlenwasserstoffgemisch (KG2b) siedet höher als (KG2a). Das Kohlenwasserstoffgemisch (KG2b) ist gegenüber (KG2a) vorzugsweise an Isohexanen und/oder Cyclopentan abgereichert und an MCP und gegebenenfalls vorhandenen Cyclohexan angereichert.

Bevorzugt wird die Leichtsiederabtrennung so ausgeführt und betrieben, dass der Strom (LS1) weniger als 5 Gew.-%, besonders bevorzugt weniger als 2,5 Gew.-% MCP enthält und das Kohlenwasserstoffgemisch (KG2b) weniger als 10 Gew.-%, besonders bevorzugt weniger als 5 Gew.-% Isohexane enthält.

Der Strom (LS1) kann beispielsweise in einen Steamcracker als sogenannter Cocrackfeed eingeleitet werden. Gegebenenfalls kann im Rahmen der Leichtsiederabtrennung ein weiterer Strom abgezogen werden, der gegenüber dem Strom (LS1) an Isohexanen abgereichert und an leichter als die Isohexane siedenden Komponenten angereichert ist, wie z. B. chlorierte Paraffine mit < 4 Kohlenstoffatomen je Molekül.

Bevorzugt ist auch eine Ausführungsform, bei der vorzugsweise ein aus Schritt f) stammender Strom (LS2) gemäß nachfolgender Beschreibung vollständig oder teilweise in oder vor (D3) zurückgeführt wird.

In Schritt e) des erfindungsgemäßen Verfahrens erfolgt die Isomerisierung des Kohlenwasserstoffgemisches (KG2a) in Gegenwart eines Katalysators, bevorzugt einer sauren ionischen Flüssigkeit unter Erhalt eines Kohlenwasserstoffgemisches (KG3), das eine gegenüber (KG2a) erhöhte Menge an Cyclohexan aufweist. Aufgrund von Schritt e) wird im erfindungsgemäßen Verfahren also MCP zu Cyclohexan isomerisiert. Sofern der optionale Schritt d) erfindungsgemäß durchgeführt wird, erfolgt die Isomerisierung sinngemäß mit dem Kohlenwasserstoffgemisch (KG2b) statt mit (KG2a).

Die Isomerisierung von MCP zu Cyclohexan gemäß Schritt e) erfolgt in Gegenwart eines Katalysators. Als Katalysatoren eignen sich prinzipiell alle dem Fachmann hierfür bekannten Katalysatoren, beispielsweise Friedel-Crafts-Katalysatoren gemäß US-A 2,846,485 wie Aluminiumchlorid, das zusätzlich HCl enthalten kann, oder Metallhalogeniden gemäß US-A 3,311,667 wie Aluminiumchlorid, Zirkoniumchlorid oder Bortrifluorid. Weiterhin eignen sich als Katalysatoren auch die in EP-A 1 995 297 verwendeten Zeolithe oder ionische Flüssigkeiten wie sie beispielsweise in WO 2011/069929 verwendet werden.

Im Rahmen der vorliegenden Erfindung erfolgt die Isomerisierung vorzugsweise in Gegenwart einer sauren ionischen Flüssigkeit mit der Zusammensetzung $K1Al_nX_{(3n+1)}$, wobei K1 ein einwertiges Kation, X gleich Halogen und 1 < n < 2,5 ist. Beispielsweise können Gemische aus zwei oder mehr sauren ionischen Flüssigkeiten eingesetzt werden, vorzugsweise wird eine saure ionische Flüssigkeit eingesetzt.

K1 ist vorzugsweise ein unsubstituiertes oder zumindest teilweise alkyliertes Ammoniumion oder ein heterocyclisches (einwertiges) Kation, insbesondere ein Pyridiniumion, ein Imidazoliumion, ein Pyridaziniumion, ein Pyrazoliumion, ein Imidazoliniumion, ein Thiazoliumion, ein Triazoliumion, ein Pyrrolidiniumion, ein Imidazolidiniumion oder ein Phosphoniumion. X ist vorzugsweise Chlor oder Brom.

Mehr bevorzugt enthält die saure ionische Flüssigkeit als Kation ein zumindest teilweise alkyliertes Ammoniumion oder ein heterocyclisches Kation und/oder als Anion ein Chloroaluminat mit der Zusammensetzung $Al_nCl_{(3n+1)}$ mit 1 < n < 2,5. Vorzugsweise enthält das zumindest teilweise alkylierte Ammoniumion einen, zwei oder drei Alkylreste mit (jeweils) ein bis zehn Kohlenstoffatomen. Sofern zwei oder drei Alkylsubstituenten mit den entsprechenden Ammoniumionen vorhanden sind, kann die jeweilige Kettenlänge unabhängig voneinander gewählt werden, vorzugsweise weisen alle Alkylsubstituenten die gleiche Kettenlänge auf. Besonders bevorzugt sind trialkylierte Ammoniumionen mit einer Kettenlänge von ein bis drei Kohlenstoffatomen. Das heterocyclische Kation ist vorzugsweise ein Imidazoliumion oder ein Pyridiniumion.

Besonders bevorzugt enthält die saure ionische Flüssigkeit als Kation ein zumindest teilweise alkyliertes Ammoniumion und als Anion ein Chloroaluminat mit der Zusammensetzung $Al_nCl_{(3n+1)}$ mit 1 < n < 2,5. Beispiele für solche besonders bevorzugten sauren ionischen Flüssigkeiten sind Trimethylammoniumchloroaluminat und Triethylammoniumchloroaluminat.

Weiterhin kann bei der Isomerisierung zusätzlich zur sauren ionischen Flüssigkeit auch ein Wasserstoffhalogenid (HX) als Cokatalysator verwendet werden. Als Wasserstoffhalogenid (HX) können prinzipiell alle denkbaren Wasserstoffhalogenide eingesetzt werden, beispielsweise Fluorwasserstoff (HF), Chlorwasserstoff (HCl), Bromwasserstoff (HBr) oder Iodwasserstoff (HI). Gegebenenfalls können die Wasserstoffhalogenide auch als Gemisch eingesetzt werden, vorzugsweise wird im Rahmen der vorliegenden Erfindung jedoch nur ein Wasserstoffhalogenid eingesetzt. Vorzugsweise wird das Wasserstoffhalogenid verwendet, dessen Halogenidteil auch in der vorstehend beschriebenen sauren ionischen Flüssigkeit (zumindest teilweise) im entsprechenden Anion enthalten ist. Vorzugsweise ist das Wasserstoffhalogenid (HX) Chlorwasserstoff (HCl) oder Bromwasserstoff (HBr). Besonders bevorzugt ist das Wasserstoffhalogenid (HX) Chlorwasserstoff (HCl).

Als Vorrichtung (IV) zur Durchführung der Isomerisierung können prinzipiell alle dem Fachmann für einen solchen Zweck bekannten Vorrichtungen verwendet werden. Vorzugsweise ist die Vorrichtung (IV) ein Rührkessel oder eine Rührkesselkaskade. Rührkesselkaskade bedeutet, dass zwei oder mehr, beispielsweise drei oder vier, Rührkessel hintereinander (in Reihe) geschaltet sind.

Die Isomerisierung wird bevorzugt bei einer Temperatur zwischen 0 °C und 100 °C, besonders bevorzugt bei einer Temperatur zwischen 30 °C und 60 °C durchgeführt. Weiterhin ist es bevorzugt, dass der Druck bei der Isomerisierung zwischen 1 und 20 bar abs. (absolut), bevorzugt zwischen 2 und 10 bar abs., beträgt.

Die Durchführung der Isomerisierung von MCP gemäß Schritt e) in Gegenwart einer sauren ionischen Flüssigkeit als Katalysator sowie gegebenenfalls einem Wasserstoffhalogenid als Cokatalysator ist dem Fachmann bekannt. Vorzugsweise bilden die Kohlenwasserstoffe (also MCP, Cyclohexan und gegebenenfalls sonstige in (KG2a) enthaltenen Kohlenwasserstoffe) und die ionische Flüssigkeit bei der Isomerisierung jeweils eine separate Phase aus, wobei Teilmengen der ionischen Flüssigkeit in der Kohlenwasserstoffphase und Teilmengen der Kohlenwasserstoffe in der ionischen Flüssigkeitsphase enthalten sein können. Sofern vorhanden, wird das Wasserstoffhalogenid, insbesondere Chlorwasserstoff, vorzugsweise gasförmig in die Vorrichtung (IV) zur Durchführung der Isomerisierung eingeleitet. Das Wasserstoffhalogenid kann, zumindest in Teilmengen, in den beiden vorgenannten Flüssigkeitsphasen sowie in einer, vorzugsweise zusätzlich vorhandenen, gasförmigen Phase enthalten sein.

Vorzugsweise wird die Isomerisierung in der Vorrichtung (IV) so durchgeführt, dass in einem Rührkessel oder einer Rührkesselkaskade zwei flüssige Phasen und eine Gasphase vorliegen. Die erste flüssige Phase enthält zu mindestens 90 Gew.-% die saure ionische Flüssigkeit und die zweite flüssige Phase enthält zu mindestens 90 Gew.-% die Kohlenwasserstoffe. Die Gasphase enthält zu mindestens 90 Gew.-% mindestens ein Wasserstoffhalogenid, vorzugsweise Chlorwasserstoff. Gegebenenfalls kann auch noch eine feste Phase vorhanden sein, die Komponenten in fester Form enthält, aus denen die ionische Flüssigkeit gebildet wird, wie beispielsweise $AlCl_3$. Dabei werden Druck und Zusammensetzung der Gasphase so eingestellt, dass der Partialdruck des gasförmigen Wasserstoffhalogenid, insbesondere von HCl-Gas, in der Gasphase zwischen 0,5 und 20 bar abs. (absolut), bevorzugt zwischen 1 und 10 bar abs. beträgt.

Weiterhin ist es im Rahmen der vorliegenden Erfindung bevorzugt, dass die Isomerisierung in einer Dispersion (D1) durchgeführt wird, wobei in der Dispersion (D1) die Phase (B) in der Phase (A) dispergiert ist, das Volumenverhältnis der Phase (A) zu Phase (B) im Bereich von 2,5 bis 4 zu 1 [Vol/Vol] liegt, die Phase (A) zu > 50 Gew.-% mindestens eine saure ionische Flüssigkeit enthält und die Phase (B) zu > 50 Gew.-% mindestens einen nichtaromatischen Kohlenwasserstoff enthält. Darüber hinaus ist es bevorzugt, dass die Dispersion (D1) zusätzlich HCl enthält und/oder gasförmiges HCl in die Dispersion (D1) eingeleitet wird.

Wie vorstehend bereits ausgeführt, wird in der Isomerisierung in Gegenwart einer sauren ionischen Flüssigkeit und gegebenenfalls eines Wasserstoffhalogenids (HX) MCP zu Cyclohexan isomerisiert bzw. (zumindest teilweise) chemisch umgesetzt. Gegebenenfalls können noch weitere in (KG2a) oder gegebenenfalls in (KG2b) enthaltene Kohlenwasserstoffe außer MCP isomerisiert werden. Die bei der Isomerisierung erhaltenen Kohlenwasserstoffe sind in dem Kohlenwasserstoffgemisch (KG3) enthalten. Das Gemisch (KG3) unterscheidet sich also hinsichtlich der Zusammensetzung und/oder Menge der darin enthaltenen Kohlenwasserstoffe von dem entsprechenden Kohlenwasserstoffgemisch (KG2a) oder gegebenenfalls (KG2b), das vor der Isomerisierung vorliegt. Die Kohlenwasserstoffgemische (KG2a) und (KG2b) wurden bereits vorstehend definiert. Allerdings sind alle Komponenten der Kohlenwasserstoffgemische (KG2a) und (KG2b), die in Schritt e) nicht isomerisiert werden, ebenfalls im Kohlenwasserstoffgemisch (KG3) enthalten.

Da bei solchen Isomerisierungsprozessen die durchzuführende Isomerisierung meist nicht zu 100 % (also vollständig) abläuft, ist im Produkt in aller Regel auch der Kohlenwasserstoff, mit dem die Isomerisierung durchgeführt wurde (in geringerer Menge als vor der Isomerisierung), noch enthalten. Da im vorliegenden Fall MCP zu Cyclohexan isomerisiert wird, ist im Isomerisierungsprodukt in der Regel ein Gemisch aus Cyclohexan und (in geringerer Menge als vor der Isomerisierung) MCP enthalten.

Das Kohlenwasserstoffgemisch (KG3) enthält vorzugsweise Cyclohexan, MCP und gegebenenfalls nicht-cyclische $C_5$-$C_6$-Alkane (Leichtsieder) und/oder gegebenenfalls schwerer als Cyclohexan siedende Komponenten. Bei den im Kohlenwasserstoffgemisch (KG3) optional noch enthaltenen Leichtsiedern und/oder Schwersiedern kann es sich um Verbindungen handeln, die im ursprünglich eingesetzten Kohlenwasserstoffgemisch (KG1) enthalten gewesen sind und in den vorausgegangenen Verfahrensschritten, insbesondere in den Schritten c) und d), nicht vollständig aus den entsprechenden Kohlenwasserstoffgemischen abgetrennt worden sind. Weiterhin kann es sich dabei auch um Verbindungen handeln, die bei der Hydrierung gemäß Schritt a) und/oder bei der Isomerisierung gemäß Schritt e) vorzugsweise als Nebenprodukte entstanden sind. Die in (KG3) optional enthaltenen schwerer als Cyclohexan siedenden Komponenten können auch Bestandteile der im Anschluss an die Hydrierung gemäß Schritt a) gegebenenfalls noch vorhandenen Restmenge an Aromaten oder Olefinen enthalten. Besonders bevorzugt enthält das Kohlenwasserstoffgemisch (KG3) Cyclohexan, Methylcyclopentan (MCP), maximal 0,1 Gew.-% an Aromaten und mindestens einen weiteren Kohlenwasserstoff ausgewählt aus n-Hexan und iso-Hexanen.

In Schritt f) des erfindungsgemäßen Verfahrens erfolgt die Isolierung von Cyclohexan aus dem Kohlenwasserstoffgemisch (KG3). Vorzugsweise wird Schritt f) in einer Rektifikationskolonne (D4) durchgeführt, wobei in (D4) aus dem Kohlenwasserstoffgemisch (KG3) ein Strom (LS2) enthaltend MCP und gegebenenfalls nichtcyclische $C_5$-$C_6$-Alkane abgetrennt und der Strom (LS2) ganz oder teilweise nach Schritt d) oder nach Schritt e) zurückgeführt wird.

Die Isolierung des Cyclohexans kann nach dem Fachmann bekannten Methoden beispielsweise unter Verwendung von einer oder mehreren Rektifikationskolonnen erfolgen, in die der Austrag der Vorrichtung eingeleitet wird, in der die Isomerisierung gemäß Schritt e) durchgeführt wurde. In der Regel wird im erfindungsgemäßen Verfahren gemäß Schritt f) -im Anschluss an die Isomerisierung gemäß Schritt e)-Cyclohexan in einer Reinheit von mindestens 98 Gew.-%, vorzugsweise von mindestens 99,5 Gew.-%, besonders bevorzugt von mindestens 99,9 Gew.-% isoliert.

Vorzugsweise wird Schritt f) des erfindungsgemäßen Verfahrens so durchgeführt, dass das Kohlenwasserstoffgemisch (KG3) enthaltend Cyclohexan, MCP, gegebenenfalls nicht-cyclische $C_5$-$C_6$-Alkane und gegebenenfalls schwerer als Cyclohexan siedende Komponenten in eine Rektifikationskolonne (D4) eingespeist wird, wobei aus (D4) an einer Entnahmestelle oberhalb des Zulaufs, bevorzugt über Kopf, der Großteil des im Zulauf zu (D4) enthaltenen MCP und gegebenenfalls an nicht-cyclischen $C_5$-$C_6$-Alkanen abgetrennt wird. Sofern in (KG3) nicht-cyclische $C_5$-$C_6$-Alkane vorhanden sind, handelt es sich dabei vorzugsweise um n-Hexan und iso-Hexane. Dieser den Großteil an MCP (und gegebenenfalls an nicht-cyclischen $C_5$-$C_6$-Alkanen) enthaltende Strom wird nachfolgend auch als Strom (LS2) bezeichnet.

Der Strom (LS2) ist weiterhin dadurch charakterisiert, dass er (bezogen auf (KG3)) an MCP angereichert und an Cyclohexan abgereichert ist, wobei dieser Strom (LS2) bevorzugt weniger als 20 Gew.-%, bevorzugt weniger als 10 Gew.-%, besonders bevorzugt weniger als 7 Gew.-% Cyclohexan enthält.

Der Strom (LS2) wird weiterhin vorzugsweise ganz oder teilweise nach Schritt d) oder nach Schritt e) zurückgeführt, vorzugsweise wird der Strom (LS2) vollständig zurückgeführt. Die Rückführung des Stroms (LS2) nach optionalem Schritt d) oder nach Schritt e) wird in der Regel so durchgeführt, dass der Strom (LS2) in oder vor die entsprechenden Vorrichtungen zur Durchführung dieser Verfahrensschritte, rückgeführt wird. Der Strom (LS2) kann also in oder vor die Vorrichtung zur Durchführung der Leichtsiederabtrennung nach Schritt d) rückgeführt werden und/oder der Strom (LS2) kann in oder vor die Vorrichtung zur Durchführung der Isomerisierung nach Schritt e) rückgeführt werden. Sofern der Strom (LS2) vor die Vorrichtung zur Durchführung der Leichtsiederabtrennung rückgeführt wird, bedeutet dies, dass der Strom (LS2) in das Kohlenwasserstoffgemisch (KG2a) außerhalb der Rektifikationsvorrichtung (D3), in der der optionale Schritt d) durchgeführt wird, eingeleitet wird. Sofern der Strom (LS2) vor die Vorrichtung zur Durchführung der Isomerisierung rückgeführt wird, bedeutet dies, dass der Strom (LS2) in das Kohlenwasserstoffgemisch (KG2a) oder gegebenenfalls (KG2b), sofern der optionale Schritt d) durchgeführt wird, außerhalb der Vorrichtung (V), in der die Isomerisierung gemäß Schritt e) durchgeführt wird, eingeleitet wird.

Insbesondere wird der Strom (LS2) nach Schritt d) zurückgeführt, vorzugsweise wird der Strom (LS2) in das Kohlenwasserstoffgemisch (KG2a) vor der Rektifikationsvorrichtung (D3), in der Schritt d) durchgeführt wird, eingeleitet.

Das Cyclohexan kann aus der Rektifikationskolonne (D4), vorzugsweise sofern keine schwerer als Cyclohexan siedenden Komponenten in die jeweilige Spezifikation beeinträchtigender Konzentration vorliegen, in einer Reinheit von mindestens 98 Gew.-%, vorzugsweise von mindestens 99,5 Gew.-%, besonders bevorzugt von mindestens 99,9 Gew.-%, über den Sumpf von (D4) oder einen unterhalb des Zulaufs gelegenen Seitenabzug von (D4), vorzugsweise dampfförmigen Seitenabzug von (D4), abgezogen werden (Option f0)). Sofern das Cyclohexan über einen unterhalb des Zulaufs gelegenen vorzugsweise dampfförmigen Seitenabzug abgezogen wird, kann über den Sumpf von (D4) ein Schwersiederstrom (S5) abgezogen werden.

Alternativ kann auch die Option f1) verwirklicht werden, wobei der über den Sumpf von (D4) abgezogene an Cyclohexan angereicherte Strom in eine Rektifikationskolonne (D5) eingeleitet wird, wobei über den Sumpf von (D5) ein Strom (S5), enthaltend schwerer als Cyclohexan siedende Komponenten, abgetrennt wird und über eine Abnahmestelle oberhalb des Zulaufs zu (D5), bevorzugt über Kopf, Cyclohexan mit einer Reinheit von mindestens 98 Gew.-%, vorzugsweise von mindestens 99,5 Gew.-%, besonders bevorzugt von mindestens 99,9 Gew.-%, abgezogen wird.

Alternativ kann auch die Option f2) verwirklicht werden, wobei ein an Cyclohexan angereicherter Strom, der bevorzugt dampfförmig ist, über den Seitenabzug aus der Rektifikationskolonne (D4) abgetrennt wird, wobei sich der Seitenabzug vorzugsweise im Abtriebsteil von (D4) befindet und/oder der an Cyclohexan angereicherte Strom aus dem Seitenabzug von (D4) in eine bevorzugt als Rektifikationskolonne ausgeführte Vorrichtung (D6) zur weiteren Aufreinigung geleitet wird und dort über eine Abnahmestelle oberhalb des Zulaufs zu (D6), bevorzugt über Kopf, Cyclohexan mit einer Reinheit von mindestens 98 Gew.-%, vorzugsweise von mindestens 99,5 Gew.-%, besonders bevorzugt von mindestens 99,9 Gew.-%, gewonnen wird.

Dabei ist es in der Option f2) weiterhin bevorzugt, dass der Zulauf des bevorzugt dampfförmigen Stromes aus (D4) nach (D6) unterhalb des untersten Bodens, der untersten Packung oder der untersten Füllkörperschüttung von (D6) erfolgt und (D6) mit einem Kopfkondensator und teilweisem Rücklauf des aus diesem abgezogenen Kondensats, nicht aber mit einem eigenen Sumpfverdampfer betrieben wird und dass die unten in (D6) anfallende Flüssigkeit ungefähr in Höhe des Seitenabzugs in die Rektifikationskolonne (D4) zurückgeleitet wird. In dieser Ausführungsform wird ein schwerer als Cyclohexan siedende Komponenten enthaltender Strom (S5) über den Sumpf von (D4) abgezogen.

Alternativ kann auch die Option f3) verwirklicht werden, wobei die Rektifikationskolonne (D4) als Trennwandkolonne ausgeführt ist, die Trennwand sich teilweise unterhalb der Zulaufstelle befindet, eine Abzugsstelle im Bereich der Trennwand liegt und über diese Abzugsstelle ein bevorzugt flüssiger Cyclohexan-Strom mit einer Reinheit von mindestens 98 Gew.-%, vorzugsweise von mindestens 99,5 Gew.-%, besonders bevorzugt von mindestens 99,9 Gew.-% entnommen wird. In dieser Ausführungsform wird ebenfalls ein schwerer als Cyclohexan siedende Komponenten enthaltender Strom (S5) über den Sumpf von (D4) abgezogen.

In Figur 4 wird Schritt f) des erfindungsgemäßen Verfahrens gemäß der vorstehend beschriebenen Option f1) nochmals verdeutlicht. CH bedeutet Cyclohexan, C6 bedeutet nicht-cyclische $C_5$-$C_6$-Alkane, insbesondere iso-Hexane und die in Klammer gesetzten Ausdrücke geben die für das Verfahren relevantesten und/oder die Hauptkomponenten des jeweiligen Stroms an. In der Ausführungsform gemäß Figur 4 wird ein Kohlenwasserstoffgemisch (KG3) eingesetzt, in dem Cyclohexan, MCP, nicht-cyclische $C_5$-$C_6$-Alkane, insbesondere iso-Hexane, und Schwersieder mit einem Normalsiedepunkt > 84 °C enthalten sind. Aus dem Sumpf von D4 wird ein an Cyclohexan angereicherter Strom (S4) in die Rektifikationskolonne (D5) eingeleitet, aus der über Kopf spezifikationsgerechtes Cyclohexan isoliert wird. Der Sumpfstrom (S5) umfasst schwerer als Cyclohexan siedende Komponenten..

Gegebenenfalls werden im Rahmen der vorliegenden Erfindung im Anschluss an die Isomerisierung gemäß Schritt e) und vor einer destillativen Abtrennung/Isolierung des Cyclohexans gemäß Schritt f) zusätzliche Aufreinigungsschritte mit dem Austrag der Isomerisierung durchgeführt. Bei diesen Aufreinigungsschritten kann es sich beispielsweise um eine neutrale und/oder alkalische Wäsche handeln, die einstufig oder mehrstufig durchgeführt werden kann. Zusätzlich oder alternativ zur Wäsche können auch spezielle Vorrichtungen, beispielsweise Destillations- oder Rektifikationsvorrichtungen, verwendet werden, um beispielsweise vorhandenes Wasserstoffhalogenid von den Kohlenwasserstoffen abzutrennen. Solche Vorrichtungen umfassen auch Vorrichtungen zur einstufigen Verdampfung, insbesondere zur Flash-Verdampfung. Zusätzlich oder alternativ können im Falle der Verwendung von saurer ionischer Flüssigkeit auch Phasentrenneinheiten, vorzugsweise Phasenscheider, den vorgenannten speziellen Vorrichtungen vorgeschaltet werden, insbesondere um die saure ionische Flüssigkeit von den Kohlenwasserstoffen abzutrennen.

In einer besonders bevorzugt Ausführungsform wird der Austrag aus der Isomerisierung in eine Phasentrenneinrichtung wie z. B. einen Phasenscheider geführt, wo eine Auftrennung in eine zu mindestens 90 Gew.-% aus saurer ionischer Flüssigkeit bestehende Phase und eine zu mindestens 90 Gew.-% aus Kohlenwasserstoffen bestehende Phase durchgeführt wird. Die zu mindestens 90 Gew.-% aus saurer ionischer Flüssigkeit bestehende Phase wird zumindest teilweise in die Isomerisierung zurückgeführt und der zu mindestens 90 Gew.% aus Kohlenwasserstoffen bestehende Phase wird, nachdem ihr gegebenenfalls in einer Destillations- oder Rektifikationsvorrichtung leichtflüchtige Bestandteile wie z. B. HCl entzogen worden sind, in eine neutrale und/oder alkalische Wäsche geführt, wo Reste der ionischen Flüssigkeit oder Bestandteile derselben wie z. B. HCl oder $AlCl_3$ entfernt werden.

In Figur 1 wird das erfindungsgemäße Verfahren in seiner Grundform unter Berücksichtigung der Schritte a) bis f) nochmals verdeutlicht, wobei aber keine Leichtsiederabtrennung gemäß optionalem Schritt d) durchgeführt wird. Ein sinngemäßes Verfahren unter Berücksichtigung des optionalen Schrittes d) wird als bevorzugte Ausführungsform in Figur 2 dargestellt. In beiden Figuren gilt Folgendes: CH bedeutet Cyclohexan, B bedeutet Benzol, HR bedeutet Hydrierreaktor und IV bedeutet Isomerisierungsvorrichtung. Die Isomerisierung wird vorzugsweise in einem Rührkessel oder einer Rührkesselkaskade durchgeführt. Das Kohlenwasserstoffgemisch (KG1) enthält Benzol, MCP, DMP und mindestens einen Leichtsieder. Sofern (KG1) zusätzlich Cyclohexan enthält, wird dies zusammen mit DMP über den Strom (S1) aus dem Verfahren abgezogen. In diesem Fall kann das Cyclohexan und/oder das bei der Hydrierung gemäß Schritt a) anfallende Cyclohexan wieder zurückgewonnen werden, wie es nachfolgend anhand der bevorzugten Ausführungsform in Verbindung mit Figur 3 verdeutlicht wird. Sinngemäß - wie vorstehend bei Figur 4 aufgeführt - sind auch in den Figuren 1 bis 3 in den Klammern nur die jeweils wichtigsten Bestandteile der entsprechenden Ströme oder Gemische aufgeführt.

Aus dem Isomerisierungsprodukt gemäß Schritt e) wird anschließend gemäß Schritt f) Cyclohexan isoliert, beispielsweise unter Verwendung von einer oder mehreren Rektifikationskolonnen, in die der Austrag der Isomerisierungsvorrichtung (IV) eingeleitet wird; dort wird Cyclohexan von nicht umgesetztem MCP und gegebenenfalls weiteren Komponenten abgetrennt, wobei vorzugsweise - wie in Figur 2 dargestellt - der an MCP angereicherte und an Cyclohexan abgereicherte Teilstrom (LS2) nach Schritt d) und/oder nach Schritt e) zurückgeführt wird. Bevorzugt wird (LS2) - wie in Figur 2 dargestellt - nach Schritt d) zurückgeleitet, insbesondere durch Einleitung in das Kohlenwasserstoffgemisch (KG2a) vor die Rektifikationsvorrichtung (D3). Die Option der Rückleitung von (LS2) nach Schritt e) ist in Figur 2 durch die gestrichene Linie (Rückleitung in die Isomerisierungsvorrichtung (IV)) angedeutet. Schritt f) ist in Figur 1 vereinfacht durch die Rektifikationsvorrichtung (D4) dargestellt. Vorzugsweise wird Schritt f) so durchgeführt, wie vorstehend im Zusammenhang mit Figur 4 dargestellt. Demzufolge ist in Figur 1 die optionale Abtrennung von schwerer als Cyclohexan siedenden Komponenten über den Strom (S5) als mögliche Variante durch den gestrichelten Pfeil angedeutet.

Das im erfindungsgemäßen Verfahren in Schritt a) eingesetzte Kohlenwasserstoffgemisch (KG1) kann aus beliebigen Quellen stammen. So ist es denkbar, dass (KG1) vor der Durchführung des erfindungsgemäßen Verfahrens aus den einzelnen Komponenten zusammengemischt wird oder dass ein Kohlenwasserstoffgemisch (KG1) durch Vereinigung von mehreren einzelnen Gemischen hergestellt wird. Vorzugsweise stammt im Rahmen der vorliegenden Erfindung das Kohlenwasserstoffgemisch (KG1) ganz oder teilweise aus einem Steamcrackverfahren.

Weiterhin ist es bevorzugt, dass (KG1) in einer Vorrichtung zur Aromatenabtrennung, die einem Steamcrackverfahren nachgeschaltet ist, aus einem aus dem Steamcrackverfahren stammenden Strom (S6) gewonnen wird. Dies bedeutet, dass im Rahmen der vorliegenden Erfindung das Kohlenwasserstoffgemisch (KG1) vorzugsweise aus einer Vorrichtung zur Aromatenabtrennung gewonnen wird.

Vorrichtungen zur Aromatenabtrennung als solche sind dem Fachmann bekannt, sie können beispielsweise eine, zwei oder noch mehr miteinander verschaltete Rektifikationsvorrichtungen umfassen. Vorzugsweise wird die Aromatenabtrennung als eine Aromaten-Extraktivrektifikation durchgeführt, insbesondere als eine Benzol-Extraktivrektifikation. Wie vorstehend bereits erwähnt, können aber gegebenenfalls eine Teilmenge des Kohlenwasserstoffgemisches (KG1) und/oder einzelne darin enthaltene Komponenten aus einer anderen Quelle als der Vorrichtung zur Aromatenabtrennung stammen. Beispielsweise können diese Teilmengen und/oder einzelnen Komponenten dem in der Vorrichtung zur Aromatenabtrennung erhaltenen Kohlenwasserstoffgemisch (KG1) anschließend zugegeben werden.

Die Vorrichtung zur Aromatenabtrennung wiederum ist bevorzugt einem Steamcrackverfahren nachgeschaltet. Ein aus dem Steamcrackverfahren stammender Strom (S6) wird in die Vorrichtung zur Aromatenabtrennung eingespeist. In der Vorrichtung zur Aromatenabtrennung wird der Strom (S6) in einen aromatenreichen Strom (S7) sowie in das Kohlenwasserstoffgemisch (KG1) aufgetrennt.

Die Durchführung eines Steamcrackverfahren als solchem ist dem Fachmann bekannt. Vorzugsweise handelt es sich im Rahmen der vorliegenden Erfindung bei dem Steamcrackverfahren um einen Naphtha-Cracker (Naphtha-Steamcrackverfahren). Der Strom (S6) stammt also vorzugsweise aus einem Naphtha-Cracker und/oder der Strom (S6) umfasst Pyrolysebenzin oder einen aus dem Pyrolysebenzin abgetrennten Teilstrom.

Der Strom (S6) wird auch als Zustrom (S6) zur Vorrichtung zur Aromatenabtrennung bezeichnet. Der Strom (S6) umfasst das Kohlenwasserstoffgemisch (KG1) und zusätzlich einen Anteil an Aromaten. Diese zusätzlichen Aromaten werden in der Vorrichtung zur Aromatenabtrennung also vom Kohlenwasserstoffgemisch (KG1) abgetrennt. Dies bedeutet weiterhin, dass das Kohlenwasserstoffgemisch (KG1) eine geringere Konzentration an Aromaten aufweist als der Zustrom (S6) zur Vorrichtung zur Aromatenabtrennung, beispielsweise kann das Kohlenwasserstoffgemisch (KG1) eine um mindestens 50% geringere Konzentration an Aromaten aufweisen als der Zustrom (S6) zur Vorrichtung zur Aromatenabtrennung.

Insbesondere wenn der Strom (S6) Pyrolysebenzin oder einen aus dem Pyrolysebenzin abgetrennten Teilstrom umfasst, kann im Rahmen der vorliegenden Erfindung der Aromatentrennung auch eine Auftrennung in jeweils an Benzol, Toluol und Xylole angereicherte Fraktionen, gegebenenfalls ergänzt durch weitere Verfahrensschritte, vorgeschaltet sein. In diesem Fall ist die an Benzol angereicherte Fraktion als Strom (S6) zu verstehen.

Die an Benzol angereicherte Fraktion wird dann bevorzugt mittels Extraktivrektifikation, beispielsweise unter Verwendung von N-Formyl-Morpholin als Hilfsstoff, in einen Benzol in hoher Reinheit enthaltenden Strom und einen an Benzol abgereicherten Strom, der auch als C6-Nichtaromatenstrom (C6-NA) bezeichnet wird, aufgetrennt. In diesem Fall ist das Kohlenwasserstoffgemisch (KG1) gemäß der vorliegenden Erfindung mit dem C6-Nichtaromatenstrom (C6-NA) gleichzusetzen.

C6-NA kann enthalten:

- lineare offenkettige und/oder verzweigte sowie zyklische (naphthenische) C5-Kohlenwasserstoffe wie z.B. n-Pentan, Isopentane, Cyclopentan,
- lineare offenkettige und/oder verzweigte sowie zyklische (naphthenische) C6-Kohlenwasserstoffe wie z.B. n-Hexan, Isohexane, Methylcyclopentan (MCP),
- lineare offenkettige und/oder verzweigte sowie zyklische (naphthenische) C7-Kohlenwasserstoffe wie z.B. n-Heptan, Isoheptane wie z.B. Dimethylpentane (DMP), Methylcyclohexan (MCP),
- Olefine und/oder Aromaten, deren Struktur von einer oder mehreren der vorgenannten Kohlenwasserstoffe mittels Eliminierung von Wasserstoff abgeleitet ist, wie z.B. Benzol oder Cyclohexen.

In einer bevorzugten Ausführungsform des erfindungsgemäßen Verfahrens wird der gemäß Schritt c) erhaltene Strom (S1) in eine Rektifikationsvorrichtung (D2) eingeleitet, wobei in (D2) Cyclohexan von DMP abgetrennt wird. Die Ausführungsform kann auch dann durchgeführt werden, wenn im in Schritt a) eingesetzten Kohlenwasserstoffgemisch (KG1) (kein) Cyclohexan enthalten ist, sondern das Cyclohexan nur durch Hydrierung von Benzol gemäß Schritt a) erhalten wird.

In dieser Ausführungsform des erfindungsgemäßen Verfahrens wird das im Strom (S1) enthaltene Cyclohexan von DMP sowie von den sonstigen im Strom (S1) gegebenenfalls enthaltenen Komponenten, beispielsweise von den Schwersiedern abgetrennt. Die konkrete Zusammensetzung des Stroms (S1) wurde vorstehend im Zusammenhang mit dem erfindungsgemäßen Schritt c) bereits beschrieben.

Die Rektifikation bzw. die Rektifikationsvorrichtung (D2) kann einstufig oder mehrstufig, beispielsweise zweistufig oder dreistufig sein, vorzugsweise ist sie dreistufig. Unter der Anzahl der Stufen (Stufigkeit) wird in diesem Zusammenhang die Anzahl der Kolonnen, jeweils inklusive Nebenapparate wie z. B. Sumpfverdampfer und Kondensatoren, verstanden, die zusammengefasst die Rektifikationsvorrichtung (D2) ausbilden. Eine dreistufige Rektifikationsvorrichtung (D2) bedeutet also, dass insgesamt drei Kolonnen, jeweils inklusive Nebenapparate wie z. B. Sumpfverdampfer und Kondensatoren, in denen jeweils ein Rektifikationsprozess durchgeführt werden kann, gemeinsam die Rektifikationsvorrichtung (D2) ausbilden. Vorzugsweise umfasst (D2) eine Extraktivrektifikationskolonne. Weiterhin ist es bevorzugt, dass der aus (D2) abgezogene an Cyclohexan angereicherte Strom höchstens 0,1 Gew.-%, vorzugsweise höchstens 0,02 Gew.-% DMP, besonders bevorzugt höchstens 0,015 Gew.-% 2,4-DMP enthält.

Sofern die Rektifikationsvorrichtung (D2) eine Extraktivrektifikationskolonne umfasst, erfolgt die Extraktivrektifikation bevorzugt unter Verwendung eines Extraktiv-Hilfsmittels (Extraktiv-Hilfsstoffs). Als Extraktiv-Hilfsmittel werden in der Regel Verbindungen eingesetzt, für die die nachfolgende Formel (1) gilt:

$$\gamma_{DMP,E}^{\infty} \Big/ \gamma_{CH,E}^{\infty} > n \qquad (1)$$

mit

$\gamma_{DMP,E}^{\infty}$ = Aktivitätskoeffizient von 2,4-Dimethylpentan im Extraktiv-Hilfsmittel bei unendlicher Verdünnung,

$\gamma_{CH,E}^{\infty}$ = Aktivitätskoeffizient von Cyclohexan im Extraktiv-Hilfsmittel bei unendlicher Verdünnung,
n = bevorzugt 1,1, besonders bevorzugt 1,3.

Als Extraktiv-Hilfsmittel werden bevorzugt sauerstoffhaltige offenkettige oder cyclische organische Verbindungen mit einem Siedepunkt mindestens 5 K oberhalb desjenigen des Cyclohexans (81 °C) eingesetzt, insbesondere solche, die eine Amid-Funktion R-CO-NR'R" als Strukturelement enthalten, wobei R, R' und R" (unabhängig voneinander) bevorzugt ausgewählt sind aus $C_1$-$C_{30}$-Alkyl oder H. Besonders geeignet als Extraktiv-Hilfsmittel sind N-Methylpyrrolidon, N-Formylmorpholin. Geeignet sind aber auch Verbindungen wie Sulfolan, Dimethylsulfoxid oder andere dem Fachmann als nichtprotische polare Lösungsmittel bekannte Verbindungen. Geeignet sind auch Mischungen mehrerer der genannten Verbindungen untereinander oder mit Wasser.

Vorzugsweise umfasst die Cyclohexan/DMP-Trennung die nachfolgenden Schritte i) bis iii), wobei die Rektifikationsvorrichtung (D2) durch die drei Komponenten (D2-1) bis (D2-3) ausgebildet wird:

i) eine Rektifikationskolonne (D2-1), in der der Großteil der Schwersieder mit einem Normalsiedepunkt > 84 °C (bezogen auf die Menge im Zulauf zu (D2-1) über Sumpf und der Großteil des Cyclohexans und sonstiger Verbindungen mit einem Normalsiedepunkt von 79 bis 84 °C (bezogen auf die Menge im Zulauf zu D2-1) über Kopf abgetrennt werden,

ii) eine Extraktivrektifikationskolonne (D2-2), in der das Kopfprodukt aus (D2-1) mit einem Extraktiv-Hilfsmittel zusammengeführt und derart destilliert wird, dass der Großteil des Extraktiv-Hilfsmittels und des Cyclohexans über Sumpf und der Großteil der im Kopfprodukt aus (D2-1) enthaltenen sonstigen Verbindungen mit einem Normalsiedepunkt von 79 bis 84 °C über Kopf aus (D2-2) abgezogen werden, und

iii) eine Regenerationskolonne (D2-3), in der der Großteil des im Sumpfstrom aus (D2-2) enthaltenen Cyclohexans über Kopf und der Großteil des im Sumpfstrom aus (D2-2) enthaltenen Extraktiv-Hilfsmittel über Sumpf abgezogen werden.

Im Rahmen der vorstehenden Schritte i) bis iii) bedeutet die Angabe "über Sumpf" eine Abnahmestelle unterhalb des Zulaufs, bevorzugt den Sumpf und die Angabe "über Kopf" eine Abnahmestelle oberhalb des Zulaufs, bevorzugt den Kopf der jeweiligen Kolonne.

Gegebenenfalls kann in dieser Ausführungsform im Zusammenhang mit den Schritten i) bis iii) ein optionaler Schritt iv) durchgeführt werden, der wie folgt definiert ist:

iv) gegebenenfalls eine Hydrierungsvorrichtung, in die entweder der Strom (S1) oder das Kopfprodukt aus (D2-3) geführt wird.

Vorzugsweise wird diese Ausführungsform jedoch ohne den optionalen Hydrierschritt iv) durchgeführt.

Der in der vorstehenden Ausführungsform enthaltene optionale Schritt iv) wird in der Regel nur dann durchgeführt, wenn im Strom (S1) ungesättigte Verbindungen enthalten sind, die somit auch in die Rektifikationsvorrichtung (D2) eingespeist werden und die zudem nicht über den Sumpf der Rektifizierkolonne (D2-1) aus dem Verfahren ausgeschleust werden. Die Hydrierung gemäß optionalem Schritt iv) kann sinngemäß zu der Hydrierung gemäß des vorstehend beschriebenen Schritt a) durchgeführt werden, bevorzugt erfolgt sie einstufig. Die Hydrierungsvorrichtung kann gegebenenfalls auch der Rektifikationsvorrichtung (D2) vorgeschaltet sein. In diesem Fall wird der Strom (S1) zunächst in die Rektifikationsvorrichtung geführt, anschließend wird der hydrierte Strom (S1) in die Rektifikationsvorrichtung (D2), insbesondere in die Rektifizierkolonne (D2-1), eingeleitet. Dies ist eine vorteilhafte Variante, wenn in Strom (S1) Komponenten wie z. B. ungesättigte Kohlenwasserstoffe enthalten sind, die mit den über den Sumpf von (D2-1) zu ziehenden Komponenten Azeotrope bilden.

Gegebenenfalls kann die vorstehend beschriebene bevorzugte Ausführungsform der Cyclohexan-/DMP-Trennung auch ohne Rektifizierkolonne (D2-1) als zwingender Bestandteil durchgeführt werden. Bei dieser Variante erfolgt die Cyclohexan-/DMP-Trennung nur unter Verwendung der beiden Kolonnen (D2-2) und (D2-3) sinngemäß, wobei gegebenenfalls auch eine Hydrierungsvorrichtung nachgeschaltet sein kann. Diese Variante wird vorzugsweise dann durchgeführt, wenn im Strom (S1) kein oder nur ein geringer Anteil an Schwersiedern mit einem Normalsiedepunkt > 84 °C enthalten ist.

Die vorstehend beschriebene bevorzugte Ausführungsform unter Verwendung der Extraktivrektifikationskolonne (D2-2) wird bevorzugt so ausgeführt und betrieben, dass der über Kopf aus (D2-2) abgezogen DMP-haltige Strom weniger als 50 Gew.-%, bevorzugt weniger als 10 Gew.-% Cyclohexan enthält. Weiterhin enthält der über Kopf Regenerationskolonne (D2-3) abgezogene cyclohexanhaltige Strom vorzugsweise weniger als 1 Gew.-%, mehr bevorzugt weniger als 10 Gew.-ppm Extraktiv-Hilfsmittel und/oder weniger als 1 Gew.-%, bevorzugt weniger als 300 Gew.-ppm Dimethylpentane, besonders bevorzugt weniger als 150 Gew.-ppm 2,4-Dimethylpentan.

Weiterhin ist es bevorzugt, dass Cyclohexan in einer Reinheit von 98 Gew.-%, insbesondere mindestens 99,5 Gew.-%, aus (D2) isoliert wird. Bezüglich der Durchführung der Isolierung des Cyclohexans gelten die gleichen Überlegungen wie vorstehend im Zusammenhang mit der Isolierung des Cyclohexan gemäß Schritt f), insbesondere im Zusammenhang mit der Rektifikationsvorrichtung (D4) ausgeführt. Alternativ kann das Cyclohexan, das aus der Rektifikationsvorrichtung (D2) gemäß der vorliegenden bevorzugten Ausführungsform stammt, mit dem Cyclohexan, das bei der Isomerisierung gemäß Schritt e) hergestellt wurde, zusammengeführt werden.

Die vorstehend beschriebene bevorzugte Ausführungsform der vorliegenden Erfindung wird in einer bevorzugten Ausgestaltung in Verbindung mit Figur 3 zusätzlich verdeutlicht. In Figur 3 haben die Abkürzungen, Pfeile und sonstigen Symbole eine sinngemäße Bedeutung, die vorstehend für Figur 1 beziehungsweise in der Beschreibung dieser bevorzugten Ausführungsform ausgeführt ist. In der Ausführungsform gemäß Figur 3 wird eine Rektifikationsvorrichtung (D2), bestehend im Wesentlichen aus drei Kolonnen ((D2-1) bis D2-3)), eingesetzt. Gegebenenfalls kann dieser Ausführungsform auch zusätzlich eine Hydrierungsvorrichtung nachgeschaltet sein (nicht dargestellt in Figur 3). Die einzelnen Kolonnen können weiterhin auch Nebenapparate wie Sumpfverdampfer oder Kondensatoren umfassen, die der Übersichtlichkeit halber in Figur 3 nicht wiedergegeben sind. EHM bedeutet Extraktiv-Hilfsmittel, S > 84 bedeutet Schwersieder mit einem Normalsiedepunkt > 84 °C, 24DMP bedeutet 2,4-Dimethylpentan und die in Klammer gesetzten Ausdrücke geben die für das Verfahren relevantesten und/oder die Hauptkomponenten des jeweiligen Stroms an. 24DMP ist beispielhaft als bevorzugte Komponente der (sonstigen) Verbindungen mit einem Normalsiedepunkt von 79 bis 84 °C aufgeführt. Als Extraktiv-Hilfsmittel wird vorzugsweise N-Methyl-2-pyrrolidon (NMP) verwendet.

Der bevorzugt aus dem Sumpf der Rektifikationsvorrichtung (D1) stammende Strom (S1), der DMP, Cyclohexan und gegebenenfalls Schwersieder mit einem Normalsiedepunkt > 78 °C enthält, wird in die Rektifizierkolonne (D2-1) eingespeist. In (D2-1) erfolgt die Aufkonzentrierung des im Strom (S1) enthaltenen Cyclohexans, wobei der Strom (S3) zunächst mittels Rektifikation in einen an schwerer als Cyclohexan siedenden Komponenten (also beispielsweise 3,3-DMP sowie sonstige Schwersieder mit einem Normalsiedepunkt > 84 °C angereicherten Strom 15 und einen an schwerer als Cyclohexan siedenden Komponenten abgereicherten Strom 16 (Strom 16 enthält also Cyclohexan sowie einen Großteil der sonstigen Verbindungen mit einem Siedepunkt von 79 bis 84 °C und eine Restmenge an Schwersiedern mit einem Normalsiedepunkt > 84 °C) aufgetrennt wird. Strom 15 kann beispielsweise als Cofeed zu einem Steamcracking-Prozess geführt oder als Bestandteil von Kraftstoff-Mischungen verwendet werden.

Strom 16 wird in eine Extraktivrektifikationskolonne (D2-2) geführt. In die Extraktivrektifikationskolonne (D2-2) wird an einer Stelle oberhalb des Zulaufs von Strom 16 ein mindestens ein Extraktiv-Hilfsmittel (EHM) enthaltender Strom 17 geführt. An einer Stelle ebenfalls oberhalb des Zulaufs von Strom 16, bevorzugt oberhalb des Zulaufs von Strom 17, z. B. am Kolonnenkopf bzw. nach dem Kopfkondensator der Kolonne, wird ein Strom 18 entnommen, der gegenüber Strom 16 an DMP, insbesondere an 2,4-DMP, angereichert ist. Vorzugsweise enthält der Strom 18 einen Großteil der im Strom 16 enthaltenen sonstigen Verbindungen mit einem Normalsiedepunkt von 79 bis 84 °C, insbesondere an 2,4-DMP. Über eine Stelle unterhalb des Zulaufs von Strom 16, bevorzugt über den Kolonnensumpf, wird ein Strom 19 entnommen, enthaltend das Extraktiv-Hilfsmittel und Cyclohexan, wobei in Strom 19 das Konzentrationsverhältnis Cyclohexan/ DMP, insbesondere an Cyclohexan/2,4-DMP höher ist als im Strom 16.

Die Extraktivrektifikationskolonne (D2-2) wird vorzugsweise so ausgeführt und betrieben, dass Strom 18 höchstens 100 Gew.-ppm, bevorzugt höchstens 10 Gew.-ppm, besonders bevorzugt höchstens 1 Gew.-ppm an Extraktiv-Hilfsmittel enthält. Dies kann erreicht werden, indem der höchste Zulauf eines EHM-haltigen Stroms mindestens 5, bevorzugt mindestens 10 theoretische Trennstufen (gemäß dem Fachmann bekannter Definition) unterhalb der Abnahmestelle von Strom 18 erfolgt und/oder (D2-2) mit einem Rücklaufverhältnis von mindestens 5, bevorzugt mindestens 10 betrieben wird.

Strom 19 wird, gegebenenfalls nach einer Vorwärmung, in die Regenerationskolonne (D2-3) geführt. Aus der Regenerationskolonne (D2-3) wird ein gegenüber Strom 19 an Cyclohexan angereicherter Strom 20 und ein gegenüber Strom 19 an Cyclohexan abgereicherter Strom 21 (Strom 21 enthält vordergründig das Extraktiv-Hilfsmittel, eine Teilmenge an Cyclohexan und (gegebenenfalls) eine Restmenge an sonstigen Verbindungen mit einem Normalsiedepunkt von 79 bis 84 °C, insbesondere an 2,4-DMP) abgezogen. Aus Strom 21 wird ein Ausschleusstrom (Purgestrom) 21 a abgezweigt, der bevorzugt maximal 5 %, besonders bevorzugt maximal 1 % der Menge von Strom 21 ausmacht. Der verbleibende Strom wird, gegebenenfalls nach einer Abkühlung (die auch im Wärmeverbund mit einer Vorwärmung von Strom 19 erfolgen kann), mindestens teilweise dem Strom 17 zugeführt und/oder in der Nähe des Stroms 16 in die Extraktivrektifikationskolonne (D2-2) zurückgeführt.

Strom 20 enthält als Hauptbestandteil Cyclohexan und kann gegebenenfalls weiter aufgearbeitet werden. beispielsweise kann spezifikationsgerechtes (hochreines) Cyclohexan aus dem Strom 20 isoliert werden. Gegebenenfalls kann der Strom 20 auch mit dem Cyclohexan oder einem cyclohexanhaltigen Strom vereinigt werden, der im erfindungsgemäßen Verfahren in der Vorrichtung (IV) (gemäß dem Schritt e)) hergestellt wird.

## Patentansprüche

1. Verfahren zur Herstellung von Cyclohexan umfassend die folgenden Schritte

    a) Hydrierung eines Kohlenwasserstoffgemisches (KG1), wobei (KG1) i) Benzol, ii) Methylcyclopentan (MCP), iii) Dimethylpentane (DMP), iv) gegebenenfalls Cyclohexan und v) gegebenenfalls mindestens eine Verbindung ausgewählt aus nicht cyclischen $C_5$-Alkanen, Cyclopentan oder nicht cyclischen $C_6$-Alkanen enthält, unter Erhalt eines Kohlenwasserstoffgemisches (KG2), das eine gegenüber (KG1) erhöhte Menge an Cyclohexan aufweist,
    b) Einspeisen des Kohlenwasserstoffgemisches (KG2) in eine Rektifikationskolonne (D1),
    c) Abtrennen eines Stromes (S1) enthaltend DMP und Cyclohexan aus dem Kohlenwasserstoffgemisch (KG2) über einen Auslass der Rektifikationskolonne (D1), wobei sich der Auslass unterhalb des Zulaufs, bevorzugt am Sumpf von (D1) befindet, unter Erhalt des Kohlenwasserstoffgemisches (KG2a), das eine gegenüber (KG2) reduzierte Menge an DMP aufweist,
    d) gegebenenfalls Abtrennen von mindestens einer Verbindung ausgewählt aus nicht cyclischen $C_5$-Alkanen, Cyclopentan oder nicht cyclischen $C_6$-Alkanen aus dem Kohlenwasserstoffgemisch (KG2a) in einer Rektifikationskolonne (D3) unter Erhalt des Kohlenwasserstoffgemisches (KG2b), das eine gegenüber (KG2a) reduzierte Menge an mindestens einer Verbindung ausgewählt aus nicht cyclischen $C_5$-Alkanen, Cyclopentan oder nicht cyclischen $C_6$-Alkanen aufweist,
    e) Isomerisierung des Kohlenwasserstoffgemisches (KG2a) oder gegebenenfalls des Kohlenwasserstoffgemisches (KG2b) in Gegenwart eines Katalysators unter Erhalt eines Kohlenwasserstoffgemisches (KG3), das eine gegenüber (KG2a) oder gegebenenfalls gegenüber (KG2b) erhöhte Menge an Cyclohexan aufweist,
    f) Isolierung von Cyclohexan aus dem Kohlenwasserstoffgemisch (KG3).

2. Verfahren gemäß Anspruch 1, **dadurch gekennzeichnet, dass** in Schritt a) die Hydrierung des Kohlenwasserstoffgemisches (KG1) in Gegenwart eines Katalysators durchgeführt wird, der als Aktivmetall mindestens ein Element der 8. bis 10. Gruppe des Periodensystems der Elemente enthält, insbesondere Nickel oder Ruthenium.

3. Verfahren gemäß Anspruch 1 oder 2, **dadurch gekennzeichnet, dass** das Kohlenwasserstoffgemisch (KG2a) mindestens 95 %, vorzugsweise mindestens 98 % der im Kohlenwasserstoffgemisch (KG2) enthaltenen Teilmenge an MCP enthält und/oder dass das Kohlenwasserstoffgemisch (KG2a) höchstens 0,1 Gew.%, vorzugsweise höchstens 0,02 Gew.% (bezogen auf die Gesamtmenge an MCP in (KG2a)) DMP enthält, besonders bevorzugt enthält das Kohlenwasserstoffgemisch (KG2a) höchstens 0,015 Gew.% (bezogen auf die Gesamtmenge an MCP in (KG2a)) 2,4-DMP.

4. Verfahren gemäß einem der Ansprüche 1 bis 3, **dadurch gekennzeichnet, dass** in Schritt a) das Kohlenwaserstoffgemisch (KG1)
    als Komponente iv) Cyclohexan enthält und/oder
    als Komponente v) mindestens eine Verbindung ausgewählt aus nicht cyclischen $C_5$-Alkanen, Cyclopentan oder nicht cyclischen $C_6$-Alkanen enthält, wobei im Fall der Anwesenheit von Komponente v) in (KG1) der Schritt d) zwingend durchgeführt wird, worauf in Schritt e) das Kohlenwasserstoffgemisch (KG2b) anstelle des Kohlenwasserstoffgemisches (KG2a) eingesetzt wird.

5. Verfahren gemäß einem der Ansprüche 1 bis 4, **dadurch gekennzeichnet, dass** in Schritt e) als Katalysator eine saure ionische Flüssigkeit eingesetzt wird, die als Kation ein zumindest teilweise alkyliertes Ammoniumion oder ein heterocyclisches Kation und/oder als Anion ein Chloroaluminat mit der Zusammensetzung $Al_nCl_{(3n+1)}$ mit $1<n<2,5$ enthält.

6. Verfahren gemäß einem der Ansprüche 1 bis 5, **dadurch gekennzeichnet, dass**

    i) in Schritt f) Cyclohexan in einer Reinheit von mindestens 98 Gew.-%, vorzugsweise von mindestens 99,5 Gew. %, besonders bevorzugt von mindestens 99,9 Gew.-% isoliert wird, und/oder
    ii) Schritt f) in einer Rektifikationskolonne (D4) durchgeführt wird, wobei in (D4) aus dem Kohlenwasserstoffgemisch (KG3) ein Strom (LS2) enthaltend MCP und gegebenenfalls nicht-cyclische $C_5$-$C_6$-Alkane abgetrennt und der Strom (LS2) ganz oder teilweise nach Schritt d) oder nach Schritt e) zurückgeführt wird.

7. Verfahren gemäß einem der Ansprüche 1 bis 6, **dadurch gekennzeichnet, dass** das Kohlenwasserstoffgemisch (KG3) enthaltend Cyclohexan, MCP, gegebenenfalls nicht-cyclische $C_5$-$C_6$-Alkane und gegebenenfalls schwerer als Cyclohexan siedende Komponenten in eine Rektifikationskolonne (D4) eingespeist wird, wobei aus (D4) an einer Entnahmestelle oberhalb des Zulaufs, bevorzugt über Kopf, der Großteil des im Zulauf zu (D4) enthaltenen MCP und gegebenenfalls an nicht-cyclischen $C_5$-$C_6$-Alkanen abgetrennt wird,

8. Verfahren gemäß Anspruch 6 oder 7, **dadurch gekennzeichnet, dass** aus der Rektifikationsskolonne (D4) Cyclohexan in einer Reinheit von mindestens 98 Gew.-% über den Sumpf von (D4) oder über einen unterhalb des Zulaufs gelegenen Seitenabzug von (D4), vorzugsweise über einen dampfförmigen Seitenabzug von (D4), abgezogen wird.

9. Verfahren gemäß Anspruch 8, **dadurch gekennzeichnet, dass** der über den Sumpf von (D4) abgezogene an Cyclohexan angereicherte Strom in eine Rektifikationskolonne (D5) eingeleitet wird, wobei über den Sumpf von (D5) ein Strom (S5), enthaltend schwerer als Cyclohexan siedende Komponenten, abgetrennt wird und über eine Abnahmestelle oberhalb des Zulaufs zu (D5), bevorzugt über Kopf, Cyclohexan mit einer Reinheit von mindestens 98 Gew.-%, vorzugsweise von mindestens 99,5 Gew. %, besonders bevorzugt von mindestens 99,9 Gew.-% abgezogen wird.

10. Verfahren gemäß Anspruch 8, **dadurch gekennzeichnet, dass** ein an Cyclohexan angereicherter Strom über den Seitenabzug aus der Rektifikationsskolonne (D4) abgetrennt wird, wobei sich der Seitenabzug vorzugsweise im Abtriebsteil von (D4) befindet und/oder der an Cyclohexan angereicherte Strom aus dem Seitenabzug von (D4) in eine bevorzugt als Rektifikationskolonne ausgeführte Vorrichtung (D6) zur weiteren Aufreinigung geleitet wird und dort über eine Abnahmesstelle oberhalb des Zulaufs von (D6), bevorzugt über Kopf, Cyclohexan mit einer Reinheit von mindestens 98 Gew.-%, vorzugsweise von mindestens 99,5 Gew. %, besonders bevorzugt von mindestens 99,9 Gew.-% gewonnen wird.

11. Verfahren gemäß einem der Ansprüche 6 bis 10, **dadurch gekennzeichnet, dass** die Rektifikationskolonne (D4) als Trennwandkolonne ausgeführt ist, die Trennwand sich teilweise unterhalb der Zulaufstelle befindet, eine Abzugsstelle im Bereich der Trennwand liegt und über diese Abzugsstelle ein bevorzugt flüssiger Cyclohexan-Strom mit einer Reinheit von mindestens 98 Gew.-%, vorzugsweise von mindestens 99,5 Gew. %, besonders bevorzugt von mindestens 99,9 Gew.-% entnommen wird.

12. Verfahren gemäß einem der Ansprüche 6 bis 11, **dadurch gekennzeichnet, dass** der Strom (LS2) nach Schritt d) zurückgeführt wird, vorzugsweise wird der Strom (LS2) in das Kohlenwasserstoffgemisch (KG2a) vor der Rektifikationskolonne (D3), in der Schritt d) durchgeführt wird, eingeleitet.

13. Verfahren gemäß einem der Ansprüche 1 bis 12, **dadurch gekennzeichnet, dass** der in Schritt c) aus dem Kohlenwasserstoffgemisch (KG2) abgetrennte Strom (S1) in eine Rektifikationssvorrichtung (D2) eingeleitet wird, wobei in (D2) Cyclohexan von DMP abgetrennt wird, vorzugsweise umfasst (D2) eine Extraktivrektifikationskolonne und/oder vorzugsweise enthält der aus (D2) abgezogene an Cyclohexan angereicherte Strom höchstens 0,1 Gew.%, vorzugsweise höchstens 0,02 Gew.% DMP, besonders bevorzugt höchstens 0,015 Gew.% 2,4-DMP.

14. Verfahren gemäß Anspruch 13, **dadurch gekennzeichnet, dass** die Cyclohexan/DMP-Trennung die nachfolgenden Schritte i) bis iii) umfasst, wobei die Rektifikationsvorrichtung (D2) durch die drei Komponenten (D2-1) bis (D2-3) ausgebildet wird:

i) eine Rektifikationskolonne (D2-1), in der der Großteil der Schwersieder (bezogen auf die Menge im Zulauf zu (D2-1)) mit einem Normalsiedepunkt > 84 °C über Sumpf und der Großteil des Cyclohexans und sonstige Verbindungen mit einem Normalsiedepunkt von 79 bis 84 °C (bezogen auf die Menge im Zulauf zu D2-1) über Kopf abgetrennt werden,
ii) eine Extraktivrektifikationskolonne (D2-2), in der das Kopfprodukt aus (D2-1) mit einem Extraktiv-Hilfsmittel zusammengeführt und derart destilliert wird, dass der Großteil des Extraktiv-Hilfsmittels und des Cyclohexans über Sumpf und der Großteil der im Kopfprodukt aus (D2-1) enthaltenen sonstigen Verbindungen mit einem Normalsiedepunkt von 79 bis 84 °C über Kopf aus (D2-2) abgezogen werden und
iii) eine Regenerationskolonne (D2-3), in der der Großteil des im Sumpfstrom aus (D2-2) enthaltenen Cyclohexans über Kopf und der Großteil des im Sumpfstrom aus (D2-2) enthaltenen Extraktiv-Hilfsmittel über Sumpf abgezogen werden.

15. Verfahren gemäß Anspruch 13 oder 14, **dadurch**

**gekennzeichnet, dass** Cyclohexan, das aus der Rektifikationsvorrichtung (D2) stammt, mit dem Cyclohexan, das bei der Isomerisierung gemäß Schritt e) hergestellt wurde, zusammengeführt wird.

16. Verfahren gemäß einem der Ansprüche 1 bis 15, **dadurch gekennzeichnet, dass** das Kohlenwasserstoffgemisch (KG1) ganz oder teilweise aus einem Steamcrackverfahren stammt.

**Claims**

1. A process for preparing cyclohexane, comprising the following steps:

   a) hydrogenating a hydrocarbon mixture (HM1), (HM1) comprising i) benzene, ii) methylcyclopentane (MCP), iii) dimethylpentanes (DMP), iv) possibly cyclohexane and v) possibly at least one compound selected from acyclic $C_5$-alkanes, cyclopentane and acyclic $C_6$-alkanes, to obtain a hydrocarbon mixture (HM2) having an elevated amount of cyclohexane compared to (HM1),
   b) feeding the hydrocarbon mixture (HM2) into a rectification column (D1),
   c) removing a stream (S1) comprising DMP and cyclohexane from the hydrocarbon mixture (HM2) via an outlet of the rectification column (D1), the outlet being below the feed, preferably at the bottom of (D1), to obtain the hydrocarbon mixture (HM2a) having a reduced amount of DMP compared to (HM2),
   d) optionally removing at least one compound selected from acyclic $C_5$-alkanes, cyclopentane and acyclic $C_6$-alkanes from the hydrocarbon mixture (HM2a) in a rectification column (D3) to obtain the hydrocarbon mixture (HM2b) having a reduced amount of at least one compound selected from acyclic $C_5$-alkanes, cyclopentane and acyclic $C_6$-alkanes compared to (HM2a),
   e) isomerizing the hydrocarbon mixture (HM2a) or optionally the hydrocarbon mixture (HM2b) in the presence of a catalyst to obtain a hydrocarbon mixture (HM3) having an elevated amount of cyclohexane compared to (HM2a) or, if appropriate, compared to (HM2b),
   f) isolating cyclohexane from the hydrocarbon mixture (HM3).

2. The process according to claim 1, wherein, in step a), the hydrogenation of the hydrocarbon mixture (HM1) is performed in the presence of a catalyst comprising, as an active metal, at least one element of groups 8 to 10 of the Periodic Table of the Elements, especially nickel or ruthenium.

3. The process according to claim 1 or 2, wherein the hydrocarbon mixture (HM2a) comprises at least 95%, preferably at least 98%, of the portion of MCP present in the hydrocarbon mixture (HM2), and/or the hydrocarbon mixture (HM2a) comprises at most 0.1% by weight, preferably at most 0.02% by weight (based on the total amount of MCP in (HM2a)), of DMP, the hydrocarbon mixture (HM2a) more preferably comprising at most 0.015% by weight (based on the total amount of MCP in (HM2a)) of 2,4-DMP.

4. The process according to any of claims 1 to 3, wherein, in step a), the hydrocarbon mixture (HM1) comprises cyclohexane as component iv) and/or comprises at least one compound selected from acyclic $C_5$-alkanes, cyclopentane and acyclic $C_6$-alkanes as component v),
   where, in the case that component b) is present in (HM1), step d) is necessarily conducted, and then, in step e), the hydrocarbon mixture (HM2b) is used rather than the hydrocarbon mixture (HM2a).

5. The process according to any of claims 1 to 4, wherein the catalyst used in step e) is an acidic ionic liquid comprising, as a cation, an at least partly alkylated ammonium ion or a heterocyclic cation and/or, as an anion, a chloroaluminate ion having the composition $Al_nCl_{(3n+1)}$ where $1 < n < 2.5$.

6. The process according to any of claims 1 to 5, wherein,

   i) in step f), cyclohexane is isolated in a purity of at least 98% by weight, preferably of at least 99.5% by weight, more preferably of at least 99.9% by weight, and/or
   ii) step f) is performed in a rectification column (D4), by removing a stream (LB2) comprising MCP and possibly acyclic $C_5$-$C_6$-alkanes from the hydrocarbon mixture (HM3) in (D4) and fully or partly recycling stream (LB2) to step d) or to step e).

7. The process according to any of claims 1 to 6, wherein the hydrocarbon mixture (HM3) comprising cyclohexane, MCP, possibly acyclic $C_5$-$C_6$-alkanes and possibly higher-boiling components than cyclohexane is fed into a rectification column (D4), and the majority of the MCP and, if present, of acyclic $C_5$-$C_6$-alkanes present in the feed to (D4) is removed from (D4) at a withdrawal point above the feed, preferably via the top.

8. The process according to claim 6 or 7, wherein cyclohexane is drawn off from the rectification column (D4) in a purity of at least 98% by weight via the bottom of (D4) or via a side draw from (D4) below the feed, preferably via a vaporous side draw from (D4).

9. The process according to claim 8, wherein the cyclohexane-enriched stream drawn off via the bottom of (D4) is introduced into a rectification column (D5), and a stream (S5) comprising higher-boiling components than cyclohexane is removed via the bottom of (D5) and cyclohexane is drawn off with a purity of at least 98% by weight, preferably of at least 99.5% by weight, more preferably of at least 99.9% by weight, via a takeoff point above the feed to (D5), preferably via the top.

10. The process according to claim 8, wherein a cyclohexane-enriched stream is removed via the side draw from the rectification column (D4), the side draw preferably being in the stripping section of (D4) and/or the cyclohexane-enriched stream from the side draw of (D4) being passed into an apparatus (D6) for further purification, preferably in the form of a rectification column, and cyclohexane being obtained therein via a takeoff point above the feed of (D6), preferably via the top, with a purity of at least 98% by weight, preferably of at least 99.5% by weight, more preferably of at least 99.9% by weight.

11. The process according to any of claims 6 to 10, wherein the rectification column (D4) takes the form of a dividing wall column, the dividing wall is partly above the feed point, a draw point is in the region of the dividing wall and this draw point is used to withdraw a preferably liquid cyclohexane stream having a purity of at least 98% by weight, preferably of at least 99.5% by weight, more preferably of at least 99.9% by weight.

12. The process according to any of claims 6 to 11, wherein stream (LB2) is recycled to step d), stream (LB2) preferably being introduced into the hydrocarbon mixture (HM2a) upstream of the rectification column (D3) in which step d) is performed.

13. The process according to any of claims 1 to 12, wherein the stream (S1) removed from the hydrocarbon mixture (HM2) in step c) is introduced into a rectification apparatus (D2), cyclohexane being separated from DMP in (D2), and (D2) preferably comprising an extractive rectification column and/or the cyclohexane-enriched stream drawn off from (D2) preferably comprising at most 0.1% by weight, preferably at most 0.02% by weight, of DMP, more preferably at most 0.015% by weight of 2,4-DMP.

14. The process according to claim 13, wherein the cyclohexane/DMP separation comprises the following steps i) to iii), the rectification apparatus (D2) being formed by the three components (D2-1) to (D2-3):

i) a rectification column (D2-1) in which the majority of the high boilers (based on the amount in the feed to (D2-1)) having a standard boiling point > 84°C is removed via the bottom and the majority of the cyclohexane and other compounds having a standard boiling point of 79 to 84°C (based on the amount in the feed to D2-1) via the top,
ii) an extractive rectification column (D2-2) in which the top product from (D2-1) is combined with an extraction aid and distilled in such a way that the majority of the extraction aid and of the cyclohexane are drawn off via the bottom and the majority of the other compounds having a standard boiling point of 79 to 84°C present in the top product from (D2-1) are drawn off from (D2-2) via the top, and
iii) a regeneration column (D2-3) in which the majority of the cyclohexane present in the bottom stream from (D2-2) is drawn off via the top and the majority of the extraction aid present in the bottom stream from (D2-2) via the bottom.

15. The process according to claim 13 or 14, wherein cyclohexane which originates from the rectification apparatus (D2) is combined with the cyclohexane which has been prepared in the isomerization in step e).

16. The process according to any of claims 1 to 15, wherein the hydrocarbon mixture (HM1) originates fully or partly from a steamcracking process.

**Revendications**

1. Procédé de fabrication de cyclohexane, comprenant les étapes suivantes :

a) l'hydrogénation d'un mélange d'hydrocarbures (KG1), (KG1) contenant i) du benzène, ii) du méthylcyclopentane (MCP), iii) des diméthylpentanes (DMP), iv) éventuellement du cyclohexane et v) éventuellement au moins un composé choisi parmi les alcanes en $C_5$ non cycliques, le cyclopentane ou les alcanes en $C_6$ non cycliques, pour obtenir un mélange d'hydrocarbures (KG2), qui présente une quantité élevée de cyclohexane par rapport à (KG1),
b) l'introduction du mélange d'hydrocarbures (KG2) dans une colonne de rectification (D1),
c) la séparation d'un courant (S1) contenant des DMP et du cyclohexane du mélange d'hydrocarbures (KG2) par une sortie de la colonne de rectification (D1), la sortie se trouvant en dessous de l'entrée, de préférence au fond de (D1), pour obtenir le mélange d'hydrocarbures (KG2a), qui présente une quantité réduite de DMP par rapport à (KG2),

d) éventuellement la séparation d'au moins un composé choisi parmi les alcanes en $C_5$ non cycliques, le cyclopentane ou les alcanes en $C_6$ non cycliques du mélange d'hydrocarbures (KG2a) dans une colonne de rectification (D3) pour obtenir le mélange d'hydrocarbures (KG2b), qui présente une quantité réduite par rapport à (KG2a) d'au moins un composé choisi parmi les alcanes en $C_5$ non cycliques, le cyclopentane ou les alcanes en $C_6$ non cycliques,

e) l'isomérisation du mélange d'hydrocarbures (KG2a) ou éventuellement du mélange d'hydrocarbures (KG2b) en présence d'un catalyseur pour obtenir un mélange d'hydrocarbures (KG3), qui présente une quantité élevée de cyclohexane par rapport à (KG2a) ou éventuellement par rapport à (KG2b),

f) l'isolement de cyclohexane à partir du mélange d'hydrocarbures (KG3).

2. Procédé selon la revendication 1, **caractérisé en ce qu'**à l'étape a), l'hydrogénation du mélange d'hydrocarbures (KG1) est réalisée en présence d'un catalyseur qui contient en tant que métal actif au moins un élément des groupes 8 à 10 du tableau périodique des éléments, notamment le nickel ou le ruthénium.

3. Procédé selon la revendication 1 ou 2, **caractérisé en ce que** le mélange d'hydrocarbures (KG2a) contient au moins 95 %, de préférence au moins 98 %, de la quantité partielle de MCP contenue dans le mélange d'hydrocarbures (KG2) et/ou **en ce que** le mélange d'hydrocarbures (KG2a) contient au plus 0,1 % en poids, de préférence au plus 0,02 % en poids (par rapport à la quantité totale de MCP dans (KG2a)) de DMP, le mélange d'hydrocarbures (KG2a) contenant de manière particulièrement préférée au plus 0,015 % en poids (par rapport à la quantité totale de MCP dans (KG2a)) de 2,4-DMP.

4. Procédé selon l'une quelconque des revendications 1 à 3, **caractérisé en ce qu'**à l'étape a), le mélange d'hydrocarbures (KG1) contient en tant que composant iv) du cyclohexane et/ou en tant que composant v) au moins un composé choisi parmi les alcanes en $C_5$ non cycliques, le cyclopentane ou les alcanes en $C_6$ non cycliques, en cas de la présence du composant v) dans (KG1), l'étape d) étant obligatoirement réalisée, le mélange d'hydrocarbures (KG2b) étant alors utilisé à la place du mélange d'hydrocarbures (KG2a) à l'étape e).

5. Procédé selon l'une quelconque des revendications 1 à 4, **caractérisé en ce qu'**à l'étape e), un liquide ionique acide est utilisé en tant que catalyseur, qui contient en tant que cation un ion ammonium au moins partiellement alkylé ou un cation hétérocyclique et/ou en tant qu'anion un ion chloroaluminate de composition $Al_nCl_{(3n+1)}$ avec $1 < n < 2,5$.

6. Procédé selon l'une quelconque des revendications 1 à 5, **caractérisé en ce que**

i) à l'étape f), du cyclohexane est isolé en une pureté d'au moins 98 % en poids, de préférence d'au moins 99,5 % en poids, de manière particulièrement préférée d'au moins 99,9 % en poids, et/ou
ii) l'étape f) est réalisée dans une colonne de rectification (D4), un courant (LS2) contenant du MCP et éventuellement des alcanes en $C_5$-$C_6$ non cycliques étant séparé du mélange d'hydrocarbures (KG3) dans (D4), et le courant (LS2) étant recyclé en totalité ou en partie après l'étape d) ou après l'étape e).

7. Procédé selon l'une quelconque des revendications 1 à 6, **caractérisé en ce que** le mélange d'hydrocarbures (KG3) contenant du cyclohexane, du MCP, éventuellement des alcanes en $C_5$-$C_6$ non cycliques et éventuellement des composants ayant un point d'ébullition plus élevé que le cyclohexane est introduit dans une colonne de rectification (D4), la majeure partie du MCP et éventuellement des alcanes en $C_5$-$C_6$ non cycliques contenus dans l'alimentation de (D4) étant séparée dans (D4) à un emplacement de soutirage au-dessus de l'alimentation, de préférence par la tête.

8. Procédé selon la revendication 6 ou 7, **caractérisé en ce que** du cyclohexane est soutiré de la colonne de rectification (D4) en une pureté d'au moins 98 % en poids par le fond de (D4) ou par une sortie latérale de (D4) disposée en dessous de l'alimentation, de préférence par une sortie latérale sous forme vapeur de (D4).

9. Procédé selon la revendication 8, **caractérisé en ce que** le courant enrichi en cyclohexane soutiré par le fond de (D4) est introduit dans une colonne de rectification (D5), un courant (S5), contenant des composants ayant un point d'ébullition plus élevé que le cyclohexane, étant séparé par le fond de (D5), et du cyclohexane ayant une pureté d'au moins 98 % en poids, de préférence d'au moins 99,5 % en poids, de manière particulièrement préférée d'au moins 99,9 % en poids, étant séparé par un emplacement de soutirage au-dessus de l'alimentation de (D5), de préférence par la tête.

10. Procédé selon la revendication 8, **caractérisé en ce qu'**un courant enrichi en cyclohexane est séparé par la sortie latérale de la colonne de rectification (D4), la sortie latérale se trouvant de préférence dans la zone de rectification de (D4) et/ou le courant enrichi en cyclohexane issu de la sortie latérale de (D4) étant introduit dans un dispositif (D6) de préférence configuré sous la forme d'une colonne de rectification pour une purification supplémentaire, et du cyclohexane ayant une pureté d'au moins 98 % en poids, de préférence d'au moins 99,5 % en poids, de manière particulièrement préférée d'au moins 99,9 % en poids, y étant obtenu par un emplacement de soutirage au-dessus de l'alimentation de (D6), de préférence par la tête.

11. Procédé selon l'une quelconque des revendications 6 à 10, **caractérisé en ce que** la colonne de rectification (D4) est configurée sous la forme d'une colonne à paroi de séparation, la paroi de séparation se trouve en partie en dessous de l'emplacement d'alimentation, un emplacement de soutirage est situé dans la zone de la paroi de séparation et un courant de cyclohexane de préférence liquide ayant une pureté d'au moins 98 % en poids, de préférence d'au moins 99,5 % en poids, de manière particulièrement préférée d'au moins 99,9 % en poids, est soutiré par cet emplacement de soutirage.

12. Procédé selon l'une quelconque des revendications 6 à 11, **caractérisé en ce que** le courant (LS2) est recyclé après l'étape d), le courant (LS2) étant de préférence introduit dans le mélange d'hydrocarbures (KG2a) avant la colonne de rectification (D3) dans l'étape d).

13. Procédé selon l'une quelconque des revendications 1 à 12, **caractérisé en ce que** le courant (S1) séparé du mélange d'hydrocarbures (KG2) à l'étape c) est introduit dans un dispositif de rectification (D2), le cyclohexane étant séparé des DMP dans (D2), (D2) comprenant de préférence une colonne de rectification extractive et/ou le courant enrichi en cyclohexane soutiré de (D2) contenant de préférence au plus 0,1 % en poids, de préférence au plus 0,02 % en poids de DMP, de manière particulièrement préférée au plus 0,015 % en poids de 2,4-DMP.

14. Procédé selon la revendication 13, **caractérisé en ce que** la séparation du cyclohexane/DMP comprend les étapes i) à iii) suivantes, le dispositif de rectification (D2) étant formé par les trois composants (D2-1) à (D2-3) :

 i) une colonne de rectification (D2-1), dans laquelle la majeure partie des composants de point d'ébullition élevé (par rapport à la quantité dans l'alimentation de (D2-1)) ayant un point d'ébullition normal > 84 °C est séparée par le fond et la majeure partie du cyclohexane et des autres composés ayant un point d'ébullition normal de 79 à 84 °C (par rapport à la quantité dans l'alimentation de D2-1) est séparée par la tête,

 ii) une colonne de rectification extractive (D2-2), dans laquelle le produit de tête de (D2-1) est réuni avec un adjuvant extractif et distillé de sorte que la majeure partie de l'adjuvant extractif et du cyclohexane soit soutirée par le fond et la majeure partie des autres composés ayant un point d'ébullition normal de 79 à 84 °C contenus dans le produit de tête de (D2-1) soit soutirée par la tête de (D2-2), et

 iii) une colonne de régénération (D2-3), dans laquelle la majeure partie du cyclohexane contenu dans le courant de fond de (D2-2) est séparée par la tête et la majeure partie de l'adjuvant extractif contenu dans le courant de fond de (D2-2) est séparée par le fond.

15. Procédé selon la revendication 13 ou 14, **caractérisé en ce que** le cyclohexane qui est issu du dispositif de rectification (D2) est réuni avec le cyclohexane qui a été fabriqué lors de l'isomérisation selon l'étape e).

16. Procédé selon l'une quelconque des revendications 1 à 15, **caractérisé en ce que** le mélange d'hydrocarbures (KG1) est issu en totalité ou en partie d'un procédé de vapocraquage.

**Fig. 1**

**Fig. 2**

Fig. 3

Fig. 4

## IN DER BESCHREIBUNG AUFGEFÜHRTE DOKUMENTE

### In der Beschreibung aufgeführte Patentdokumente

- US 20030109767 A
- EP 1403236 A
- US 20050082201 A
- WO 2010027987 A
- US 3311667 A
- EP 1995297 A
- EP 1992673 A
- US 2846485 A
- US 3406217 A
- US 6503465 B
- WO 2011069929 A
- WO 2011069957 A
- US 4053369 A
- US 4955468 A
- WO 0222528 A
- EP 1127601 B1

### In der Beschreibung aufgeführte Nicht-Patentliteratur

- Kirk-Othmer Encyclopedia of Chemical Technology. 17. August 2001, vol. 8, 739 ff